Europäisches Patentamt
European Patent Office
Office européen des brevets

(19)

(11) Publication number: **0 354 757 A2**

(12) **EUROPEAN PATENT APPLICATION**

(21) Application number: **89308037.4**

(22) Date of filing: **08.08.89**

(51) Int. Cl.[5]: **C 07 D 501/20**
C 07 D 501/18,
C 07 D 471/04,
C 07 D 498/04, A 61 K 31/545
//(C07D471/04,221:00,205:00),
(C07D498/04,265:00,205:00)

(30) Priority: **11.08.88 GB 8819104**

(43) Date of publication of application:
**14.02.90 Bulletin 90/07**

(84) Designated Contracting States:
**BE CH DE FR GB IT LI NL**

(71) Applicant: **BEECHAM GROUP PLC**
**Beecham House Great West Road**
**Brentford Middlesex TW8 9BD (GB)**

(72) Inventor: **Pearson, Michael John Beecham Pharmaceuticals**
**Brockham Park**
**Betchworth Surrey RH3 7AJ (GB)**

**Burton, George Beechan Pharmaceuticals**
**Brockham Park**
**Betchworth Surrey RH3 7AJ (GB)**

**Fell, Stephen Christopher Martin**
**Beecham Pharmaceuticals Brockham Park**
**Betchworth Surrey RH3 7AJ (GB)**

(74) Representative: **West, Vivien et al**
**Beecham Pharmaceuticals Great Burgh Yew Tree Bottom Road**
**Epsom Surrey KT18 5XQ (GB)**

The title of the invention has been amended (Guidelines for Examination in the EPO, A-III, 7.3).

(54) **Cephalosporins and homologous compounds, process for their preparation and pharmaceutical compositions containing them.**

(57) β-Lactam antibiotics of the formula (Ia) or pharmaceutically acceptable salts or pharmaceutically acceptable in vivo hydrolysable esters thereof, and the use thereof, are disclosed:

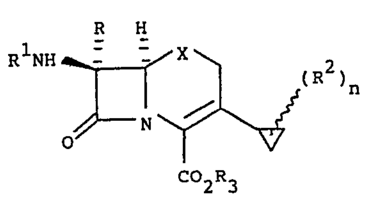

(Ia)

wherein
R is hydrogen, methoxy or formamido;
$R^1$ is an acyl group, in particular that of an antibacterially active cephalosporin;
$R^2$ is optionally substituted alkyl, acyl, alkoxycarbonyl, halogen, aryl, heterocyclyl, carboxy, carboxamido, or cyano; $CO_2R_3$ is carboxy or a carboxylate anion; X is S,SO,$SO_2$,O or $CH_2$; and n is zero or an integer from 1 to 5.
Processes for the preparation of compounds together with intermediates for use therein are disclosed.

EP 0 354 757 A2

## Description

## Novel Compounds

This invention relates to novel β-lactam containing compounds, their preparation and their use, and in particular to a novel class of cephalosporins. These compounds have antibacterial properties, and therefore are of use in the treatment of bacterial infections in humans and animals caused by a wide range of organisms.

GB-A-1 581 184 discloses a class of cephalosporin antibiotics having an optional 3-cycloalkyl substituent.

The present invention provides a compound of formula (I) or a salt thereof:

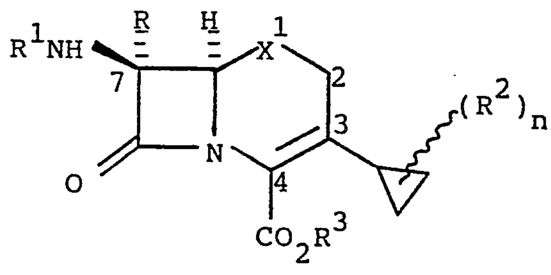

(I)

wherein

R is hydrogen, methoxy or formamido;

$R^1$ is an acyl group, in particular that of an antibacterially active cephalosporin;

$R^2$ is optionally substituted alkyl, acyl, alkoxycarbonyl, halogen, aryl, heterocyclyl, carboxy, carboxamido, or cyano; $CO_2R^3$ is carboxy or a carboxylate anion, $R^3$ is a readily removable carboxy protecting group; X is S,SO,$SO_2$,O or $CH_2$; and n is zero or an integer from 1 to 5.

It will be appreciated that when n is zero the cyclopropyl ring bonded to position 3 of the cephalosporin nucleus is unsubstituted, when n is 2 or more each $R^2$ may be the same or different and that a cyclopropyl ring carbon atom may be mono- or di-substituted. Preferably n is zero, 1 or 2.

Certain compounds of the invention have been found to posses good Gram-positive activity and oral absorption.

In compounds of formula (I) wherein R is formamido the, formamido group can exist in conformations wherein the hydrogen atoms of the -NH-CHO moiety are cis- or trans-; of these the cis conformation normally predominates,

Since the β-lactam antibiotic compounds of the present invention are intended for use as therapeutic agents in pharmaceutical compositions, it will be readily appreciated that preferred compounds within formula (I) are pharmaceutically acceptable, i.e. are compounds of formula (Ia) or pharmaceutically acceptable salts or pharmaceutically acceptable in vivo hydrolysable esters thereof:

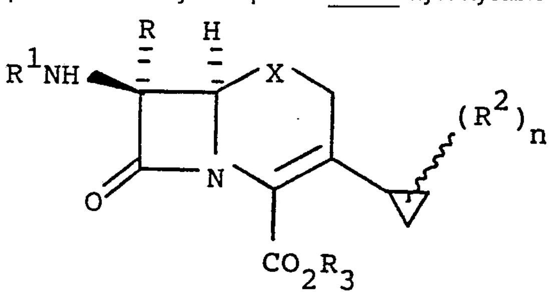

(Ia)

wherein R, $R^1$, $R^2$, X and n are as defined with respect to formula (I) and the group $CO_2R_3$ is $CO_2R^3$ where $CO_2R^3$ is carboxy or a carboxylate anion.

Those compounds of the formula (I) wherein $R^3$ is a readily removable carboxy protecting group other than a pharmaceutically acceptable in vivo hydrolysable ester, or which are in non-pharmaceutically acceptable salt form are primarily useful as intermediates in the preparation of compounds of the formula (Ia) or a pharmaceutically acceptable salt or pharmaceutically acceptable in vivo hydrolysable ester thereof.

Suitable readily removable carboxy protecting groups for the group $R^3$ include groups forming ester derivatives of the carboxylic acid, including in vivo hydrolysable esters. The derivative is preferably one which

may readily be cleaved.

It will be appreciated that also included within the scope of the invention are salts and carboxy-protected derivatives, including in vivo hydrolysable esters, of any carboxy groups that may be present as optional substituents in compounds of formula (I) or (Ia). Also included within the scope of the invention are acid addition salts of any amino group or substituted amino group that may be present as optional substituents in compounds of formula (I) or (Ia).

Suitable ester-forming carboxyl-protecting groups are those which may be removed under conventional conditions. Such groups for $R^3$ include benzyl, p-methoxybenzyl, benzoylmethyl, p-nitrobenzyl, 4-pyridylmethyl, 2,2,2-trichloroethyl, 2,2,2-tribromoethyl, t-butyl, t-amyl, allyl, diphenylmethyl, triphenylmethyl, adamantyl, 2-benzyloxyphenyl, 4-methylthiophenyl, tetrahydrofur-2-yl, tetrahydropyran-2-yl, pentachlorophenyl, acetonyl, p-toluenesulphonylethyl, methoxymethyl, a silyl, stannyl or phosphorus-containing group, an oxime radical of formula $-N=CHR^4$ where R4 is aryl or heterocyclic, or an in vivo hydrolysable ester radical such as defined below.

When used herein the term 'aryl' includes phenyl and naphthyl, each optionally substituted with up to five, preferably up to three, groups selected from halogen, mercapto, $C_{1-6}$ alkyl, phenyl, $C_{1-6}$ alkoxy, hydroxy($C_{1-6}$)alkyl, mercapto($C_{1-6}$)alkyl, halo($C_{1-6}$) alkyl, hydroxy, amino, nitro, carboxy, $C_{1-6}$ alkylcarbonyloxy, alkoxycarbonyl, formyl, or $C_{1-6}$ alkylcarbonyl groups.

The terms 'heterocyclyl' and 'heterocyclic' as used herein include aromatic and non-aromatic, single and fused, rings suitably containing up to four hetero-atoms in each ring selected from oxygen, nitrogen and sulphur, which rings may be unsubstituted or substituted by, for example, up to three groups selected from halogen, ($C_{1-6}$)alkyl, ($C_{1-6}$)alkoxy, halo($C_{1-6}$)alkyl, hydroxy, carboxy, carboxy salts. carboxy esters such as ($C_{1-6}$)alkoxycarbonyl, ($C_{1-6}$)alkoxycarbonyl($C_{1-6}$)alkyl, aryl, and oxo groups. Each heterocyclic ring suitably has from 4 to 7, preferably 5 or 6, ring atoms. A fused heterocyclic ring system may include carbocyclic rings and need include only one heterocyclic ring. Compounds within the invention containing a heterocyclyl group may occur in two or more tautometric forms depending on the nature of the heterocyclyl group; all such tautomeric forms are included within the scope of the invention.

When used herein the term 'halogen' refers to fluorine, chlorine, bromine and iodine.

When used herein in relation to variable $R^2$, the term acyl includes $C_{1-6}$ alkylcarbonyl.

When used herein the terms 'alkyl' and 'alkoxy' (or 'lower alkyl' and 'lower alkoxy') include straight and branched chain alkyl groups containing from 1 to 6 carbon atoms, such as methyl, ethyl, propyl and butyl. A particular alkyl group is methyl.

Suitable optional substituents for an alkyl group include amino, cyano, halogen, hydroxy, nitro, carboxy and salts and esters thereof.

A carboxyl group may be regenerated from any of the above esters by usual methods appropriate to the particular $R^3$ group, for example, acid- and base-catalysed hydrolysis, or by enzymically-catalysed hydrolysis, or by hydrogenolysis under conditions wherein the remainder of the molecule is substantially unaffected.

Examples of suitable pharmaceutically acceptable in vivo hydrolysable ester groups include those which break down readily in the human body to leave the parent acid or its salt. Suitable ester groups of this type include those of part formulae (i), (ii), (iii) and (iv):

and (iv):

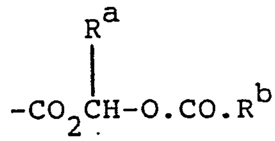

(i)

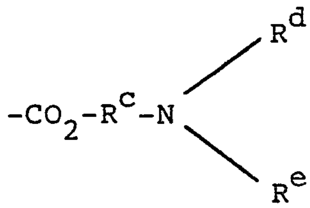

(ii)

$-CO_2CH_2-OR^f$ (iii)

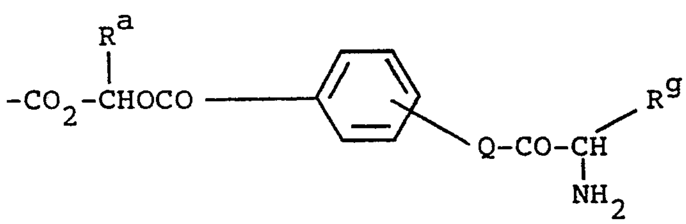

(iv)

wherein $R^a$ is hydrogen, $C_{1-6}$ alkyl, $C_{3-7}$ cycloalkyl, methyl, or phenyl, $R^b$ is $C_{1-6}$ alkyl, $C_{1-6}$ alkoxy, phenyl, benzyl, $C_{3-7}$ cycloalkyl, $C_{1-6}$ alkyl $C_{3-7}$ cycloalkyl, 1-amino $C_{1-6}$ alkyl, or 1-($C_{1-6}$ alkyl)amino $C_{1-6}$ alkyl; or $R^a$ and $R^b$ together form a 1,2-phenylene group optionally substituted by one or two methoxy groups; $R^c$ represents $C_{1-6}$ alkylene optionally substituted with a methyl or ethyl group and $R^d$ and $R^e$ independently represent $C_{1-6}$ alkyl; $R^f$ represents $C_{1-6}$ alkyl; $R^g$ represents hydrogen or phenyl optionally substituted by up to three groups selected from halogen, $C_{1-6}$ alkyl, or $C_{1-6}$ alkoxy; and Q is oxygen or NH.

Examples of suitable in vivo hydrolysable ester groups include, for example, acyloxyalkyl groups such as acetoxymethyl, pivaloyloxymethyl, α-acetoxyethyl, α-pivaloyloxyethyl, 1-(cyclohexylcarbonyloxy)prop-1- yl, and (1-aminoethyl)carbonyloxymethyl; alkoxycarbonyloxyalkyl groups, such as ethoxycarbonyloxymethyl and α-ethoxycarbonyloxyethyl; dialkylaminoalkyl especially di-loweralkylamino alkyl groups such as dimethylaminomethyl, dimethylaminoethyl, diethylaminomethyl or diethylaminoethyl; lactone groups such as phthalidyl and dimethoxyphthalidyl; and esters linked to a second β-lactam antibiotic or to a β-lactamase inhibitor.

A further suitable pharmaceutically acceptable in vivo hydrolysable ester group is that of the formula:

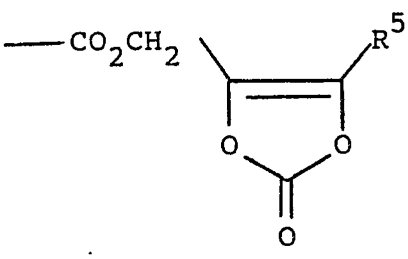

wherein $R^5$ is hydrogen, $C_{1-6}$ alkyl or phenyl.

Suitable pharmaceutically acceptable salts of the carboxy group of the compound of formula (I) include metal salts, eg aluminium, alkali metal salts such as sodium or potassium, alkaline earth metal salts such as calcium or magnesium, and ammonium or substituted ammonium salts, for example those with lower alkylamines such as triethylamine, hydroxy-lower alkylamines such as 2-hydroxyethylamine, bis-(2-hydroxyethyl)amine or tris-(2-hydroxyethyl)amine, cycloalkylamines such as dicyclohexylamine, or with procaine, dibenzylamine, N,N-dibenzylethylenediamine, 1-ephenamine, N-methylmorpholine, N-ethylpiperidine, N-benzyl-β-phenethylamine, dehydroabietylamine, N,N'-bisdehydro-abietylamine, ethylenediamine, or bases of the pyridine type such as pyridine, collidine or quinoline, or other amines which have been used to form salts with known penicillins and cephalosporins. Other useful salts include the lithium salt and silver salt. Salts within compounds of formula (I), may be prepared by salt exchange in conventional manner.

In compounds of formula (I) or (Ia), the group X may be sulphur or an oxidised sulphur atom, i.e. a sulphoxide (SO) or sulphone ($SO_2$) group. When X is a sulphoxide group it will be understood that α- and

β-isomers may exist; both such isomers are encompassed within the scope of the present invention.

Preferably X is sulphur.

Advantageously, R is hydrogen in compounds of formula (I) or (Ia).

Suitable acyl groups $R^1$ in compounds of formula (I) include those of formulae (a) - (f):

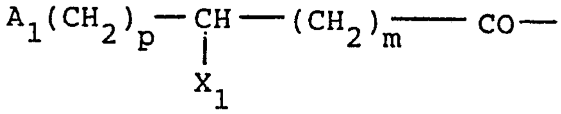

(a)

$A_2CO-$ (b)

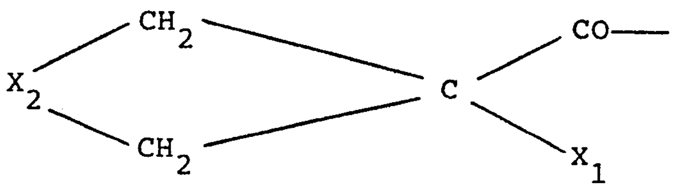

(c)

$A_2-X_3-(CH_2)_p-CO-$ (d)

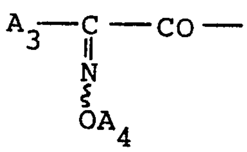

(e)

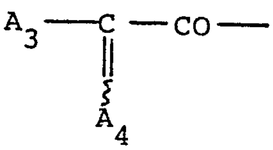

(f)

wherein p is 0, 1 or 2; m is 0, 1 or 2; $A_1$ is $C_{1-6}$ alkyl, substituted $C_{1-6}$ alkyl, $C_{3-6}$ cycloalkyl, cyclohexenyl, cyclohexadienyl, an aromatic (including heteroaromatic) group, such as phenyl, substituted phenyl, thienyl, pyridyl, or an optionally substituted thiazolyl group, a $C_{1-6}$ alkylthio group or $C_{1-6}$ alkyloxy; $X_1$ is a hydrogen or halogen atom, a carboxylic acid, carboxylic ester, sulphonic acid, azido, tetrazolyl, hydroxy, acyloxy, amino, ureido, acylamino, heterocyclylamino, guanidino or acylureido group; $A_2$ is an aromatic group, for example a phenyl, 2,6-dimethoxyphenyl,2-alkoxy-1-naphthyl, 3-arylisoxazolyl, or a 3-aryl-5-methylisoxazolyl group, such as 3-(2-chloro-6-fluorophenyl)5-methylisoxazol-4-yl; a substituted alkyl group; or a substituted dithietane; $X_2$is a $CH_2OCH_2$, $CH_2SCH_2$ or alkylene group; $X_3$ is an oxygen or sulphur atom; $A_3$ is an aryl or heteroaryl group such as phenyl, substituted phenyl or aminothiazolyl in which the amino group is optionally protected; and $A_4$ is hydrogen, $C_{1-6}$ alkyl, $C_{3-6}$ cycloalkyl, $C_{1-6}$ alkoxycarbonyl($C_{1-6}$)alkyl, $C_{2-6}$ alkenyl, carboxy($C_{1-6}$)alkyl, $C_{2-6}$ alkynyl, aryl and aryl($C_{1-6}$)alkyl.

The term 'heteroaryl' as used herein means a heteroaromatic heterocyclic ring or ring system, suitably having 5 or 6 ring atoms in each ring.

Suitably when $R^1$ is a group (a), $A_1$ is $C_{1-6}$ alkyl, $C_{3-6}$ cycloalkyl, cyclohexenyl, cyclohexadienyl, phenyl, substituted phenyl such as hydroxy-phenyl, thienyl or pyridyl; and $X_1$ is a hydrogen or halogen atom, or a carboxy, carboxylic ester, azido, tetrazolyl, hydroxy, acyloxy, optionally protected amino, ureido, guanidino or acylureido group. In compounds of formula (Ia), in a particularly preferred group (a), $A_1$ is 4-hydroxyphenyl, X is amino and p and m are 0.

Suitably when $R^1$ is a group (d), $A_2$ is phenyl, $X_2$ is oxygen and p is 0.

Alternatively, when $R^1$ is a group of formula (e), suitable values for the group $A_3$ include those commonly found in antibacterially active cephalosporins containing a hydroxyimino or substituted hydroxyimino group in the side chain attached to position 7 of the cephalosporin nudeus, for example phenyl, thien-2-yl, thien-3-yl, fur-2-yl, fur-3-yl, pyrid-2-yl, pyrid-3-yl, pyrid-4-yl, and 2-aminothiazol-4-yl in which the amino group is optionally

protected.

Preferred groups for $A_3$ include phenyl, 2-aminothiazol-4-yl, fur-2-yl, thien-2-yl, 2-(2-chloroacetamido)thiazol-4-yl, 2-tritylaminothiazol-4-yl, 3-amino-1,2,4-thiadiazol-5-yl and 4-aminopyrimid-2-yl.

In compounds of formula (Ia), a particularly preferred group for $A_3$ is 2-aminothiazol-4-yl.

Suitable values for the group A4 include hydrogen, methyl, ethyl, cyclopropylmethyl, triphenylmethyl (trityl), cyclobutyl, cyclopentyl, cyclohexyl, phenyl, carboxymethyl, t-butoxycarbonylmethyl and carboxypropyl.

Preferred values for $A_4$ in compounds of formula (Ia) include methyl, hydrogen and carboxymethyl.

It will be appreciated that compounds of the invention wherein $R^1$ is a group of formula (e) (or (f) can exist as syn and anti (or E and Z) isomers or mixtures thereof. Both isomers are encompassed within the scope of this invention.

Preferably the compounds of the invention wherein $R^1$ is a group (e) have the syn configuration (i.e. have the group $OA_4$ syn to the amide linkage) or are enriched in that isomer.

Similarly, when $R^1$ is a group (f), the group $A_4$ is preferably cis to the amide linkage, i.e. when group (f) is 2-amino-thiazol-4-yl, the Z-configuration is preferred.

Certain compounds of the invention include an amino group which may be protected. Suitable amino protecting groups are those well known in the art which may be removed under conventional conditions without disruption of the remainder of the molecule.

Examples of amino protecting groups include $C_{1-6}$ alkanoyl; benzoyl; benzyl optionally substituted in the phenyl ring by one or two substituents selected from $C_{1-4}$ alkyl, $C_{1-4}$ alkoxy, trifluoromethyl, halogen, or nitro; $C_{1-4}$ alkoxycarbonyl; benzyloxycarbonyl or trityl substituted as for benzyl above; allyloxycarbonyl,trichloroethoxycarbonyl or chloroacetyl.

Some of the compounds of this invention may be crystallised or recrystallised from solvents such as organic solvents. In such cases solvates may be formed. This invention includes within its scope stoichiometric solvates including hydrates as well as compounds containing variable amounts of water that may be produced by processes such as lyophilisation.

Since the antibiotic compounds of the invention are intended for use in pharmaceutical compositions it will readily be understood that they are each provided in substantially pure form, for example at least 60% pure, more suitably at least 75% pure and preferably at least 85%, especially at least 95% pure (% are on a weight for weight basis). Impure preparations of the compounds may be used for preparing the more pure forms used in the pharmaceutical compositions; these less pure preparations of the compounds should contain at least 1%, more suitably at least 5% and preferably from 10 to 49% of a compound of the formula (I) or salt thereof.

It will be appreciated that $R^2$ may be in either the cis or the trans configuration, and the present invention includes either isomer, as well as mixtures of both isomers. A suitable value for $R^2$ includes alkoxycarbonyl, for example methoxycarbonyl.

Specific compounds within this invention of formula (Ia) include the following and pharmaceutically acceptable salts and in-vivo hydrolysable esters thereof:

(6R,7R)-7-[2-(2-aminothiazol-4-yl)-2-(Z)-methoxyiminoacetamido]-3-cyclopropylceph-3-em-4-carboxylic acid,

(6R,7R)-7-[(2R)-2-amino-2-(4-hydroxyphenyl)acetamido]-3-cyclopropylceph-3-em-4-carboxylic acid,

(6R,7R)-7-[2-(2-aminothiazol-4-yl)-2-(Z)-carboxymethoxyiminoacetamido]-3-cyclopropylceph-3-em-4-carboxylic acid,

(6R,7R)-7-[2-(2-aminothiazol-4-yl)2-(Z)-hydroxyiminoacetamido]3-cyclopropylceph-3-em-4-carboxylic acid,

(6R,7R)-7-[2-(2-aminothiazol-4-yl)-2-(Z)-methoxyiminoacetamido]-3-[1RS,2RS)-1-methoxycarbonylcycloprop-2-yl]ceph-3-em-4-carboxylic acid,

(6R,7R)-7-[2-(2-aminothiazol-4-yl)-2-(Z)-methoxyiminoacetamido]-3-(1-methoxycarbonylcycloprop-1-yl)ceph-3-em-4-carboxylic acid, and

(6R,7R)-7-[2-(2-aminothiazol-4-yl)-2-(Z)-methoxyiminoacetamido]-3-(2RS)-1,1-bismethoxycarbonylcycloprop-2-yl]ceph-3-em-4-carboxylic acid.

The present invention further provides a process for the preparation of a compound of formula (I) which process comprises treating a compound of formula (IV) or a salt thereof:

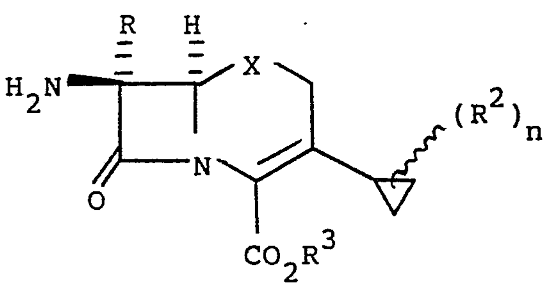

(IV)

wherein R, $R^2$, $R^3$, X and n are as hereinbefore defined, wherein any reactive groups may be protected, and wherein the amino group is optionally substituted with a group which permits acylation to take place; with an

N-acylating derivative of an acid of formula (V):
$R^1OH$ (V)
wherein $R^1$ is as defined with respect to formula (I) and wherein any reactive groups may be protected; and thereafter, if necessary or desired, carrying out one or more of the following steps:

i) removing any protecting groups;
ii) converting the group $R^3$ into a different group $R^3$;
iii) converting the group $R^1$ into a different group $R^1$;
iv) converting X into a different X;
v) converting the product into a salt.

Acids of formula (V) may be prepared by methods known in the art, or methods analogous to such processes. Suitable processes include those described, for example, in UK Patent GB 2 107 307 B, UK Patent Specification No. 1,536,281, and U.K. Patent Specification No. 1,508,064.

Suitable groups which permit acylation to take place and which are optionally present on the amino group of the starting material of the formula (IV) include N-silyl, N-stannyl and N-phosphorus groups, for example trialkylsilyl groups such as trimethylsilyl, trialkyltin groups such as tri-n-butyltin, groups of formula $-P.R^6R^7$ wherein $R^6$ is an alkyl, haloalkyl, aryl, aralkyl, alkoxy, haloalkyl, aryl, aralkyl, alkoxy, haloalkoxy, aryloxy, aralkyloxy or dialkylamino group, $R^7$ is the same as $R^6$ or is halogen or $R^6$ and $R^7$ together form a ring; suitable such phosphorus groups being $-P(OC_2H_5)_2$, $-P(C_2H_5)_2$,

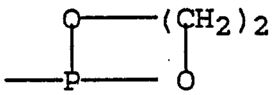 and 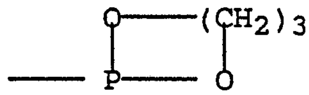

A group which may optionally be introduced onto the amino group in the compound of formula (IV) is trimethylsilyl.

Advantageously the silylation reaction may be carried out in situ prior to the acylation reaction, with a silylating agent that does not require concomitant addition of base. Suitable silylating agents include, for example, N-(trimethylsilyl)-acetamide, N,O-bis(trimethylsilyl)acetamide, N,O-bis(trimethylsilyl)trifluoroacetamide, N-methyl-N-trimethylsilylacetamide, N-methyl-N-trimethylsilyltrifluoroacetamide, N,N′-bis(trimethylsilyl)urea, and N,O-bis(trimethylsilyl)carbamate. A preferred silylating agent is N,O-bis(trimethylsilyl)acetamide. The silylation reaction may suitably be carried out in an inert, anhydrous organic solvent such as dichloromethane at room temperature or at an elevated temperature, for example 30 - 60°C, preferably 40 -50°C.

The above process may optionally be carried out in the presence of a small quantity, for example 0.1 equivalents, of a silyl halide, for example a tri($C_{1-6}$)alkylsilyl halide, especially trimethylsilyl chloride.

A reactive N-acylating derivative of the acid (V) is employed in the above process. The choice of reactive derivative will of course be influenced by the chemical nature of the substituents of the acid.

Suitable N-acylating derivatives include an acid halide preferably the acid chloride or bromide. Acylation with an acid halide may be effected in the presence of an acid binding agent for example, tertiary amine (such as pyridine or dimethylaniline), molecular sieves, an inorganic base (such as calcium carbonate or sodium bicarbonate) or an oxirane, which binds hydrogen halide liberated in the acylation reaction. The oxirane is preferably a ($C_{1-6}$)- 1,2-alkylene oxide -such as ethylene oxide or propylene oxide. The acylation reaction using an acid halide may be carried out at a temperature in the range -50°C to +50°C, preferably -20°C to +20°C, in aqueous or non-aqueous media such as water, acetone, tetrahydrofuran, ethyl acetate, dimethylacetamide, dimethylformamide, acetonitrile, dichloromethane, 1,2-dichloroethane, or mixtures thereof. Alternatively, the reaction may be carried out in an unstable emulsion of water-immiscible solvent, especially an aliphatic ester or ketone, such as methyl isobutyl ketone or butyl acetate.

The acid halide may be prepared by reacting the acid (V) or a salt or a reactive derivative thereof with a halogenating (eg chlorinating or brominating) agent such as phosphorus pentachloride, thionyl chloride, oxalyl chloride or phosgene.

Alternatively, the N-acylating derivative of the acid (V) may be a symmetrical or mixed anhydride. Suitable mixed anhydrides are anhydrides with, for example, carbonic acid monoesters, trimethyl acetic acid, thioacetic acid, diphenylacetic acid, benzoic acid, phosphorus acids (such as phosphoric, phosphorous, and phosphinic acids) or aromatic or aliphatic sulphonic acids (such as p-toluenesulphonic acid or methanesulphonic acid). When a symmetrical anhydride is employed, the reaction may be carried out in the presence of 2,6-lutidine as catalyst.

Alternative N-acylating derivatives of acid (V) are the acid azide, or activated esters such as esters with 2-mercaptopyridine, cyanomethanol, p-nitrophenol, 2,4-dinitrophenol, thiophenol, halophenols, including pentachlorophenol, monomethoxyphenol, N-hydroxy succinimide, N-hydroxybenzotriazole, or 8-hydroxyquinoline; or amides such as N-acylsaccharins, N-acylthiazolidin-2-thione or N-acylphthalimides; or an alkylidene iminoester prepared by reaction of the acid (V) with an oxime.

Other reactive N-acylating derivatives of the acid (V) include the reactive intermediates formed by reaction in situ with a condensing agent such as a carbodiimide, for example, N,N′-diethyl-, dipropyl- or diisopropylcarbodiimide, N,N′-di-cyclohexylcarbodiimide, or N-ethyl-N′-[3-(dimethylamino)propyl]carbodiimide; a suitable

carbonyl compound, for example, N,N′-carbonyldiimidazole or N,N′-carbonylditriazole; an isoxazolinium salt, for example, N-ethyl-5-phenylisoxazolinium-3-sulphonate or N-t-butyl-5-methylisoxazolinium perchlorate; or an N-alkoxycarbonyl 2-alkoxy-1,2-dihydroquinoline, such as N-ethoxycarbonyl 2-ethoxy-1,2-dihydroquinoline. Other condensing agents include Lewis acids (for example $BBr_3$ - $C_6H_6$); or a phosphoric acid condensing agent such as diethylphosphorylcyanide. The condensation reaction is preferably carried out in an organic reaction medium, for example, methylene chloride, dimethylformamide, acetonitrile, alcohol, benzene, dioxan or tetrahydrofuran.

A further method of forming the N-acylating derivative of the acid of formula (V) is to treat the acid of formula (V) with a solution or suspension preformed by addition of a carbonyl halide, preferably oxalyl chloride, or a phosphoryl halide such as phosphorus oxychloride, to a halogenated hydrocarbon solvent, preferably dichloromethane, containing a lower acyl tertiary amide, preferably N,N-dimethylformamide. The N-acylating derivative of the acid of formula (V) so derived may then be caused to react with a compound of formula (IV). The acylation reaction may conveniently be carried out at -40° to +30°C, if desired in the presence of an acid binding agent such as pyridine. A catalyst such as 4-dimethylaminopyridine may optionally also be added. A preferred solvent for the above acylation reaction is dichloromethane.

When the group X is S, SO, or $SO_2$, the group X may be converted into a different group X by methods of oxidation or reduction well known in the art of cephalosporin and penicillin synthesis, as described, for example, in European Patent Application Publication No. 0 114 752. For example, sulphoxides (in which X is SO) may be prepared from the corresponding sulphide (in which X is S) by oxidation with a suitable oxidising agent, for example an organic peracid such as m-chloroperbenzoic acid.

In the process described hereinabove, and in the processes described hereinbelow, it may be necessary to remove protecting groups. Deprotection may be carried out by any convenient method known in the art such that unwanted side reactions are minimised. Separation of unwanted by-products may be carried out using standard methods.

In a further process of the invention compounds of formula (I) in which X is S, SO or $SO_2$ may be prepared by heating an azetidinone compound of formula (VI):

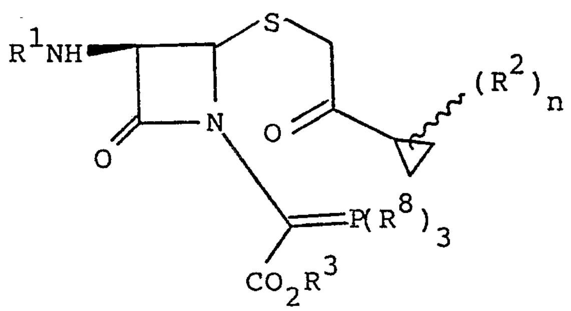

(VI)

wherein $R^1$, $R^2$, $R^3$ and n are as hereinbefore defined, $R^8$ is phenyl or n-butyl, and any reactive group may be protected; for example in xylene for approximately 70 h at 160°C;

and thereafter if necessary or desired, carrying out one or more of the following steps:

i) removing any protecting groups;
ii) converting the group $R^3$ into a different group $R^3$;
iii) converting the group $R^1$ into a different group $R^1$;
iv) converting X into a different X;
v) converting the product into a salt.

Compounds of formula (VI) may be prepared from a compound of formula (VII):

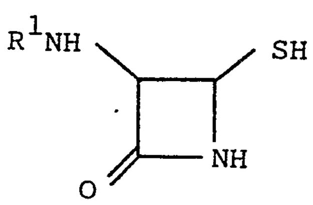

(VII)

wherein $R^1$ is as hereinbefore defined;

by following the procedure outlined in Scheme I wherein $R^1$, $R^2$, $R^3$, $R^8$ and n are as hereinbefore defined:

Scheme I

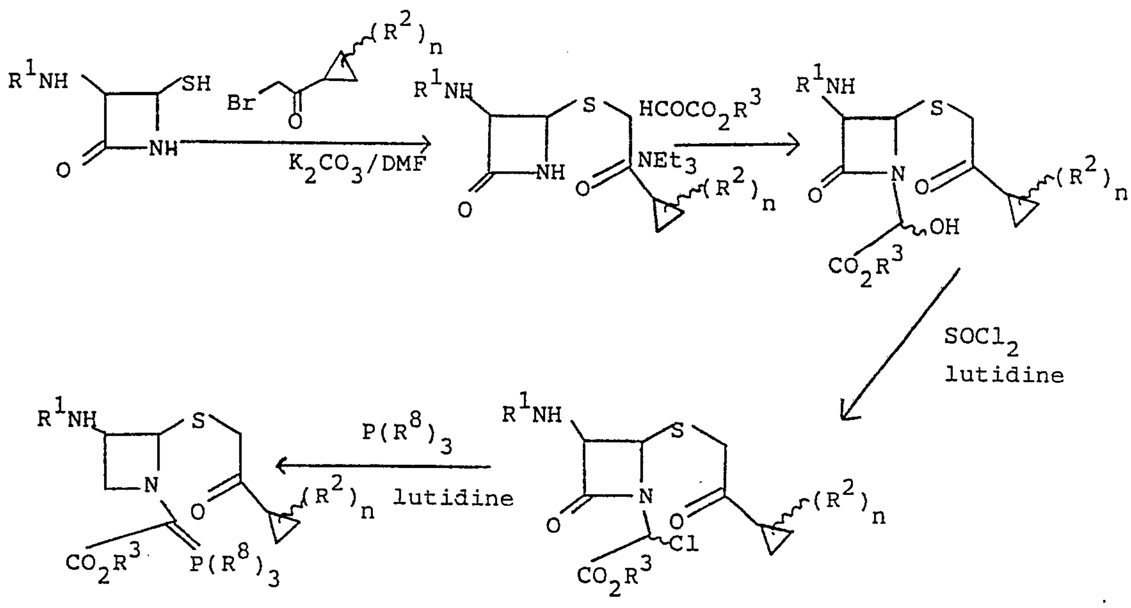


J.H.C. Nayler, N.F. Osborne, M.J. Pearson and R. Southgate, J. Chem. Soc., Perkin Trans. I, 1976, 1615.

In yet a further process of the invention, compounds of formula (I) may be prepared by heating a compound of formula (VIII):

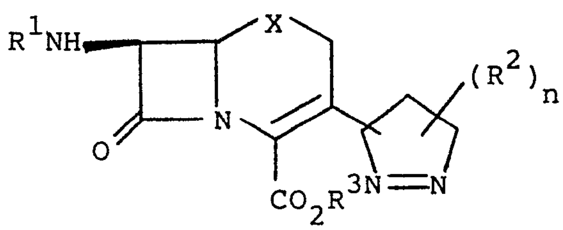

(VIII)

wherein $R^1$, $R^2$, $R^3$ and n are as hereinbefore defined, and any reactive group may be protected, for example in ethyl acetate for approximately 48 h;
and thereafter, if necessary or desired carrying out one or more of the following steps:

i) removing any protecting groups;
ii) converting the group $R^3$ into a different group $R^3$;
iii) converting the group $R^1$ into a different group $R^1$;
iv) converting X into a different X;
v) converting the product into a salt.

Compounds of formula (VIII) may be prepared from a compound of formula (IX):

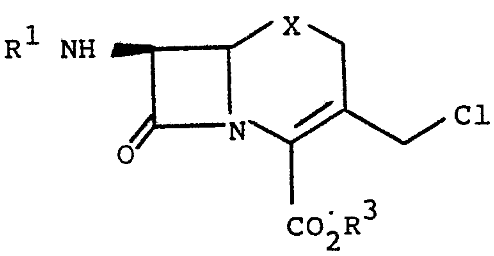

(IX)

wherein $R^1$ and $R^3$ are as hereinbefore defined; by following the procedure outlined in Scheme II, wherein $R^1$, $R^2$, $R^3$ and n are as hereinbefore defined and $R_2$ is $R^2$ or hydrogen:

Scheme II

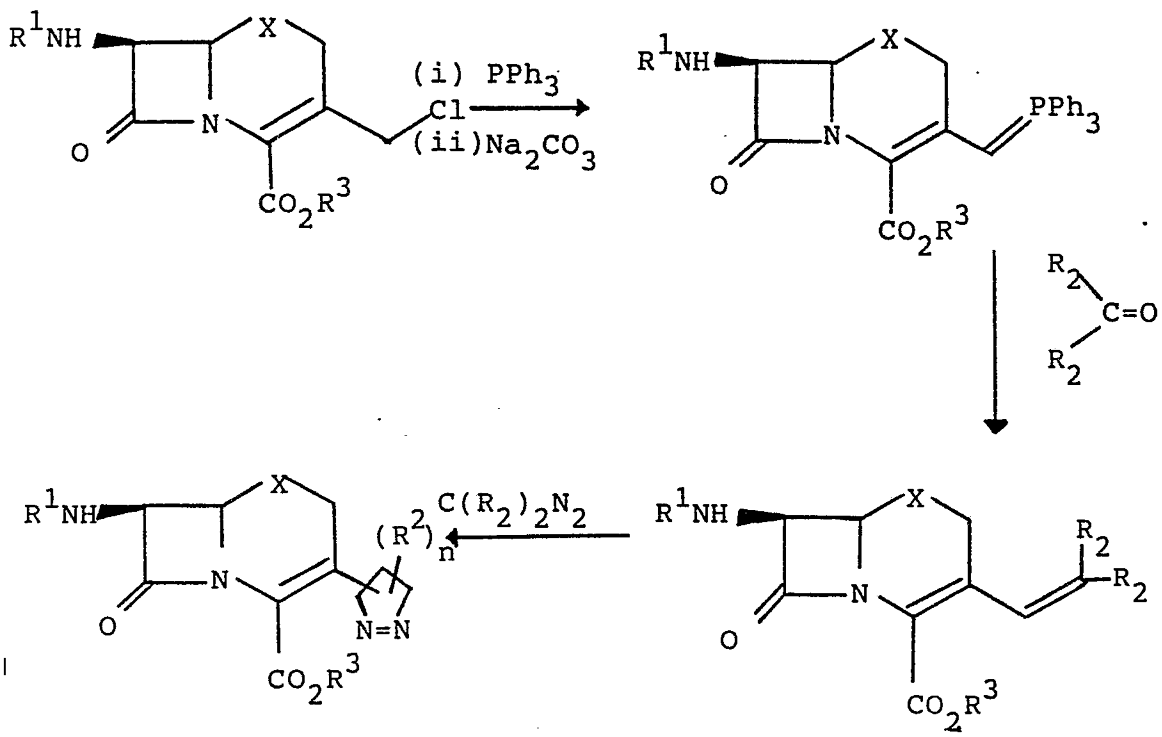


Compounds of formulae (IV), (VI) and (VIII) are new and form a further aspect of the invention.

It should be noted that in processes of this invention $\Delta^2$-cephems may function as intermediates in the synthetic sequences. Subsequent isomerisation steps by methods well known in cephalosporin chemistry will provide $\Delta^3$-cephems of the invention.

The present invention also provides a pharmaceutical composition which comprises a compound of formula (Ia) or a pharmaceutically acceptable salt or in vivo hydrolysable ester thereof and a pharmaceutically acceptable carrier. The compositions of the invention include those in a form adapted for oral, topical or parenteral use and may be used for the treatment of bacterial infection in mammals including humans.

The antibiotic compounds according to the invention may be formulated for administration in any convenient way for use in human or veterinary medicine, by analogy with other antibiotics.

The composition may be formulated for administration by any route, such as oral, topical or parenteral. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water

for injection and filter sterilised before filling into a suitable vial or ampoule and sealing. Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No toxicological effects are indicated when a compound of formula (Ia) or a pharmaceutically acceptable salt or in vivo hydrolysable ester thereof is administered in the above-mentioned dosage range.

The compound of formula (Ia) may be the sole therapeutic agent in the compositions of the invention or a combination with other antibiotics or with a β-lactamase inhibitor may be employed.

Advantageously, the compositions also comprise a compound of formula (II) or a pharmaceutically acceptable salt or ester thereof:

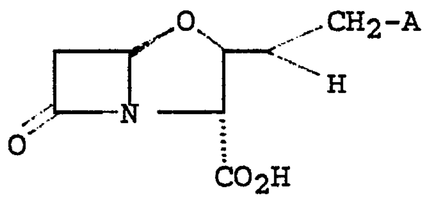

(II)

wherein
A is hydroxyl, substituted hydroxyl, thiol, substituted thiol, amino, mono- or di-hydrocarbyl-substituted amino, or mono- or di-acylamino.

A further advantageous composition comprises a compound of formula (Ia) or a pharmaceutically acceptable salt or in vivo hydrolysable ester thereof together with a compound of formula (III) or a pharmaceutically acceptable salt or in vivo hydrolysable ester thereof:

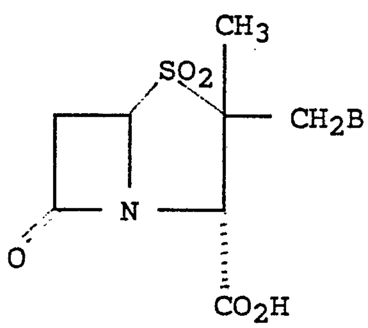

(III)

wherein
B represents hydrogen or halogen, for example chloro.

Further suitable β-lactamase inhibitors include 6β-bromopenicillanic acid and pharmaceutically acceptable salts and in vivo hydrolysable esters thereof and 6β-iodopenicillanic acid and pharmaceutically acceptable salts and in vivo hydrolysable esters thereof, as well as 6-alkylidene penems and pharmaceutically acceptable salts and in vivo hydrolysable esters thereof, as described in, for example, EP-A-0 410 768 and EP-A-0 154 132 (both Beecham Group).

Such compositions of this invention which include a β-lactamase inhibitory amount of a β-lactamase inhibitor are formulated in a conventional manner using techniques and procedures per se known in the art.

The present invention also includes a method of treating bacterial infections in humans and animals which comprises the administration of a therapeutically effective amount of an antibiotic compound of this invention of the formula (Ia) or a pharmaceutically acceptable salt or pharmaceutically acceptable in vivo hydrolysable ester thereof.

The antibiotic compounds of the present invention are active against a wide range of organisms including both Gram-negative organisms such as E.coli, in particular NCTC 10418, and Gram-positive organisms such as S. aureus, in particular S. aureus Oxford.

The following Examples illustrate the preparation of the compounds of the present invention.

Example 1

t-Butyl (6R,7R)-7-Amino-3-cyclopropylceph-3-em-4-carboxylate

a) (3R,4R)-4-Cyclopropylcarbonylmethylthio-3-phenoxyacetamidoazetidin-2-one

Trimethylsilyl trifluoromethanesulphonate (5.325ml, 27.5mmol) in dichloromethane (10ml) was added dropwise to an ice bath cooled solution of cyclopropylmethylketone (2.50ml, 25mmol) and triethylamine (3.825ml, 27.5mmol) in dichloromethane (25ml). After 0.5h N-bromosuccinimide (4.895g, 27.5mmol) was added, the mixture stirred 0.5h then washed with water (25ml) and aqueous sodium bicarbonate (25ml), dried and concentrated to provide crude bromomethylcyclopropylketone. To this in DMF (25ml) were added (3R,4R)-4-mercapto-3-phenoxyacetamidoazetidin-2-one (N.F. Osborne, J. Chem. Soc., Perkin Trans. I, 1980, 146) (6.30g, 25mmol) and potassium carbonate (3.450g, 25mmol) and the mixture stirred overnight, diluted with ethyl acetate (150ml), washed with water (2 x 100ml) and brine (50ml), dried and evaporated to a gum. Flash chromatography on silica gel eluting with 60-80% ethyl acetate in hexane provided the title compound as a crystalline solid, recrystallised from ethyl acetate-hexane (6.138g, 74%), mp 105-106°C; (Found: C, 57.44; H, 5.42; N, 8.21%. $C_{16}H_{18}N_2O_4S$ requires: C, 57.47; H, 5.43; N, 8.38%); (Found: $M^+$, 334.0981. $C_{16}H_{18}N_2O_4S$ requires M, 334.0987); $\nu_{max}$ ($CH_2Cl_2$) 3390, 1775, 1685, 1222 and 1060cm$^{-1}$; $\delta_H$ (250MHz, $(CD_3)_2SO$) 0.8-1.0 (4H, m), 2.05-2.2 (1H, m), 3.61 (2H, s), 4.58 and 4.61 (2H, ABq, J 15Hz), 4.91 (1H, d, J 4.6Hz), 5.27 (1H, dd, J 8.5, 4.3Hz), 6.9-7.35 (5H, m), 8.82 (1H, s), 9.00 (1H, d, J 8.6Hz).

b) t-Butyl (2RS)-2-[(3R,4R)-(4-Cyclopropylcarbonylmethylthio-3-phenoxyacetamidoazetidin-2-on-1-yl]-2-hydroxyacetate

(3R,4R)-4-Cyclopropylcarbonylmethylthio-3-phenoxyacetamidoazetidin-2-one (4.714g, 14.lmmol) and t-butyl glyoxylate hydrate (5.222g, 35.25mmol) in 1,2-dichloroethane (150ml) were heated under a Dean and Stark trap for 2h, cooled to ca. 25°C and triethylamine (196µL, 1.41mmol) added. The solution was stirred for 2h, concentrated and flash chromatographed on silica gel eluting with 50-60% ethyl acetate in hexane to provide the title compound as a mixture of stereoisomers (5.286g, 81%); $\nu_{max}$ ($CH_2Cl_2$) 3480, 3405, 1782, 1735, 1690, 1238 and 1155cm$^{-1}$; 6H (250MHz, $CDCl_3$) 0.9-1.15 (4H, m), 1.52 (9H, s), 1.9-2.1 (1H, m), 3.48, 3.58 (together 2H, 2s), 4.40, 4.57 (together 1H, 2d, J 7.3Hz, $D_2O$ exch.), 4.59 (2H, s), 5.03, 5.07 (together 1H, 2d, J 4.8Hz), 5.29, 5.38 (together 1H, 2d, J 7.5Hz, 2s on $D_2O$ exch.), 5.47, 5.56 (together 1H, 2dd, J 8.8, 4.9Hz), 6.9-7.4 (5H, m), 7.43, 7.59 (together 1H, 2d, J 8.8Hz); [Mass spectrum: +ve ion (3-nitrobenzylalcohol) $MH^+$ (465), $MNa^+$ (487), $2MH^+$ (929)].

c) t-Butyl 2-[(3R,4R)-4-Cyclopropylcarbonylmethylthio-3-phenoxyacetamidoazetidin-2-on-1-yl]-2-triphenylphosphorylideneacetate

Thionyl chloride (1.25ml, 17.1mmol) in THF (5ml) was added dropwise to t-butyl (2RS)-2-[(3R,4R)-4-cyclopropylcarbonylmethylthio-3-phenoxyacetamidoazetidin-2-on-1-yl]-2-hydroxyacetate (5.286g, 11.4mmol) and 2,6-lutidine (1.98ml, 17.1mmol) in THF (50ml) cooled to -10°C. The mixture was stirred 0.5h without further cooling, filtered and the filtrate evaporated to a foam. Toluene (20ml) was added then evaporated to leave crude t-butyl (2RS)-2-chloro-2-[(3R,4R)-4-cyclopropylcarbonylmethylthio-3-phenoxyacetamidoazetidin-2-on-1-yl]acetate. This in dioxan (100ml) containing triphenylphosphine (5.974g, 22.8mmol) and 2,6-lutidine (1.98ml, 17.1mmol) was stirred for 24h, diluted with ethyl acetate (200ml), washed with water (2 x 100ml) and brine (50ml), dried, concentrated and flash chromatographed on silica gel eluting with 50-80% ethyl acetate in hexane to yield the phosphorane as a foam (6.748g, 84%); $\nu_{max}$ ($CH_2Cl_2$) 3405, 1765, 1690, 1640, 1515 and 1165cm$^{-1}$. [Mass spectrum: +ve ion (3-nitrobenzyalchohol, sodium acetate) $MNa^+$ (731)].

d) t-Butyl (6R,7R,)-3-Cyclopropyl-7β-phenoxyacetamidoceph-3-em-4-carboxylate

The phosphorane (3.421g) and benzoic acid (50mg) in xylene (50ml) were heated in an oil bath at 160°C for 65h, cooled and flash chromatographed on silica gel eluting with 20-25% ethyl acetate in hexane to give the title compound (0.683g, 33%); $\nu_{max}$ ($CH_2Cl_2$) 3420, 1785, 1698. 1520, 1370. 1240 and 1158cm$^{-1}$; $\delta_H$ (90MHz, $CDCl_3$) 0.4-1.0 (4H, m), 1.52 (9H, s), 2.15-2.55 (1H, m), 2.84 and 2.99 (2H, ABq, J 17.1Hz), 6.50 (2H, s), 4.92 (1H, d, J 5.4Hz), 5.71 (1H, dd, J 9.5, 5.4Hz), 6.8-7.4 (5H, m), 7.54 (1H, d, J 9.5Hz); [Mass spectrum: +ve ion (thioglycerol) $MNa^+$ (453)].

e) t-Butyl (6R,7R)-7-Amino-3-cyclopropylceph-3-em-4-carboxylate

Phosphorous pentachloride (0.354g, 1.71mmol) was added to t-butyl (6R,7R)-3-cyclopropyl-7-phenoxyacetamidoceph-3-em-4-carboxylate (0.490g, 1.14mmol) and N-methylmorpholine (251µl, 2.28mmol) in dichloromethane (10ml) at -15°C. The mixture was stirred at -10±5°C for 0.75h, cooled to -15°C, methanol (2.5ml) added, stirred for 0.5h, water (5ml) added, stirred a further 0.5h then evaporated to small volume. The residue in ethyl acetate (20ml) and water (10ml) was adjusted to pH 7 (saturated aqueous sodium bicarbonate), the organic phase collected, washed with water (2 x 10ml) and brine (10ml), dried, concentrated and flash

chromatographed on silica gel eluting with 50-60% ethyl acetate in hexane to provide the title compound (0.212g, 63%);(Found: M+, 296.1189. $C_{14}H_{20}N_2O_3S$ requires M, 296.1195); $\nu_{max}$ ($CH_2Cl_2$) 1778, 1718, 1160 and 845cm$^{-1}$; $\delta_H$ (90MHz, $CDCl_3$) 0.4-1.0 (4H, m), 1.52 (9H, s), 1.93 (2H, br s), 2.0-2.5 (1H, m), 2.88 and 3.13 (2H, ABq, J 17.1Hz), 4.65 (1H, d, J 5Hz), 4.87 (1H, d, J 5Hz).

Example 2

Diphenylmethyl (6R,7R)-7-Amino-3-cyclopropylceph-3-em-4-carboxylate

a) Diphenylmethyl (6R,7R)-7-Phenylacetamido-3-(pyrrazolin-3-yl)ceph-3-em-4-carboxylate

Diphenylmethyl (6R,7R)-7-phenylacetamido-3-vinylceph-3-em-4-carboxylate (Hideaki Yamanaka, Toshiyuki Chiba, Kohji Kawabata, Hisashi Takasugi, Takashi Masugi and Takao Takaya, J. Antibiot., 1985, 38, 1738) (3.78g) dissolved in a minimum amount of dichloromethane was treated with an ethereal solution of diazomethane (containing ca. 0.93g, 3 equivalents) at room temperature. After 3-4h t.l.c. analysis (50% ethyl acetate/hexane) showed only trace amounts of starting material. Removal of excess diazomethane and solvent afforded a gum. Flash chromatography on silica gel eluting with 40-50% ethyl acetate/hexane gave the title compound as a pale yellow foam (2.296g, 56%); $\nu_{max}$ ($CH_2Cl_2$) 3400, 1785, 1720, 1680, 1490, and 1365cm$^{-1}$; $\delta_H$ (400MHz, $CDCl_3$) 1.12 (1H, m), 2.12 (1H, m), 2.65 and 3.58 (2H, ABq, J 19Hz), 3.61 and 3.68 (2H, ABq, J 16Hz), 4.05 (1H, m), 4.81 (1H, ddt, J 9.8, 17.6 and 2.4Hz), 5.07 (1H, d, J 4.9Hz), 5.31 (1H, br t, J 9.4Hz), 5.91 (1H, dd, J 4.9, 9.1Hz), 6.05 (1H, d, J 9.1Hz), 6.89 (1H, s), 7.24-7.41 (15H, m).

b) Diphenylmethyl (6R,7R)-7-Phenylacetamido-3-cyclopropylceph-3-em-4-carboxylate

Diphenylmethyl (6R,7R)-7-phenylacetamido-3-(pyrrazolin-3-yl)ceph-3-em-4-carboxylate (0.1g) in ethyl acetate (15ml) was heated under reflux for ca. 48h. The solvent was removed and the residual gum flash chromatographed on silica gel eluting wtih 30% ethyl acetate/hexane. The title comoound was obtained as a colourless crystalline solid (0.06g, 63%), mp 168-169°C (ethyl acetate/ hexane); (Found; M+, 524.1778. $C_{31}H_{28}N_2O_4S$ requires M, 524.1770); $\nu_{max}$ ($CH_2Cl_2$), 3400, 3300 (br), 1780, 1715, 1685, 1595, 1500 and 1380cm$^{-1}$; $\delta_H$ (250MHz, $CDCl_3$) 0.40 (1H, m), 0.50 (1H, m), 0.76 (2H, m), 2.37 and 2.73 (2H, ABq, J 16Hz), 2.68 (1H, m), 3.63 and 3.70 (2H, ABq, J 16Hz), 4.96 (1H, d, J 4.3Hz), 5.60 (1H, dd, J 4.3, 8.7Hz), 6.64 (1H, d, J 8.7Hz), 6.90 (1H, s), 7.23-7.45 (15H, m).

c) Diphenylmethyl (6R,7R)-7-Amino-3-cyclopropylceph-3-em-4-carboxylate

Diphenylmethyl (6R,7R)-7-phenylacetamido-3-cyclopropyl ceph-3-em-4-carboxylate (1.29g) in dry dichloromethane (20ml) was cooled to -20°C and treated with N-methylmorpholine (0.594g) and resublimed phosphorous pentachloride (0.672g) for 30mins. The reaction mixture was quenched with methanol (20ml) for 30mins, and then water (20ml) for a further 30mins. The dichloromethane was removed in vacuo, and ethyl acetate added. After adjusting to pH 8 with aqueous sodium bicarbonate the organic layer was separated, washed with brine and dried. Removal of solvent afforded the crude title compound, which was purified by flash chromatography on silica gel eluting with 70% ethyl acetate/hexane to give a white solid (0.806g, 81%), mp 124-126°C (ethyl acetate/hexane); $\nu_{max}$ ($CH_2Cl_2$) 3400 (w), 1770, 1715 and 1600 (w) cm$^{-1}$;$\delta_H$ (250MHz, $CDCl_3$) 0.49-0.71 (2H, m), 0.83 (1H, m), 1.78 (2H, s, exch.), 2.45 (1H, m), 2.84 and 3.00 (2H, ABq, J 17Hz), 4.67 (1H, d, J 4.7Hz), 4.94 (1H, d, J 4.7Hz), 6.97 (1H, s), 7.23-7.96 (10H, m); [Mass spectrum: $M^+$ (406)].

Example 3

Sodium (6R,7R)-7-[2-(2-Aminothiazol-4-yl)-2-(Z)-methoxyiminoacetamido]-3-cyclopropylceph-3-em-4-carboxylate

a) Diphenylmethyl (6R,7R)-7-[2-(2-Chloroacetamidothiazol-4-yl)-2(Z)-methoxyiminoacetamido]-3-cyclopropylceph-3-em-4-carboxylate

Diphenylmethyl (6R,7R)-7-amino-3-cyclopropylceph-3-em-4-carboxylate (0.406g) in dichloromethane (10ml), at 0°C was treated with pyridine (0.19g) and then 2-(2-chloroacetamidothiazol-4-yl)-2-(Z)-methoxyiminoacetyl chloride hydrochloride (0.399g). After warming to room temperature over 30mins the solution was washed with dilute hydrochloric acid, brine and then dried. Removal of solvent and trituration with ether/ethyl acetate afforded the title compound as a white solid (0.55g, 83%); $\nu_{max}$ ($CH_2Cl_2$), 1770 (shoulder), 1750, 1700 (shoulder), 1680 and 1540Cm$^{-1}$; $\delta_H$ (250MHz, $CDCl_3$) 0.55 (1H, m), 0.84 (1H, m), 2.43 and 2.72 (2H, ABq, J 16Hz), 2.77 (1H, m), 4.10 (3H, s), 4.12 and 4.26 (2H, ABq, J 16Hz), 5.11 (1H, d, J 4.2Hz), 5.68 (1H, dd, J 4.2, 8.0Hz), 6.93 (1H, s), 7.25 (1H, s), 7.27-7.44 (10H, m), 8.04 (1H, d, J 8.0Hz, exch.), 10.89 (1H, s, exch.); [Mass spectrum: +ve ion (thioglycerol) $MH^+$ (666)].

b) Sodium (6R,7R)-7-[2-(2-Aminothiazol-4-yl)-2-(Z)-methoxyiminoacetamido]-3-cyclopropylceph-3-em-4-carboxylate

Diphenylmethyl (6R,7R)-7-[2-(2-chloroacetamidothiazol4-yl)-2-(Z)-methoxyiminoacetamido]-3-cyclopropylceph3-em-4-carboxylate (0.5g) in dichloromethane (40ml) was cooled in ice/water and treated with anisole

(2.27g) and trifluoroacetic acid (TFA) (4.03g). After warming to room temperature over 1h, the solvent and excess reagents were removed in vacuo. The residue was partitioned between ethyl acetate and sat. sodium bicarbonate. The aqueous layer was washed with ethyl acetate and then treated at room temperature with sodium N-methyldithiocarbamate (0.116g). After 2h, the reaction mixture was left overnight at ca. 5°C. The aqueous solution was extracted with ethyl acetate and then concentrated to ca. 5ml. The crude sodium salt was chromatographed on HP20SS eluting with 0-4% THF in water. The fractions containing the title compound were combined, concentrated and freeze-dried to give an off-white foam (0.273g, 82%); $\lambda_{max}$ ($H_2O$) 230 (ε 13,588), 262nm (ε 12,971); $\nu_{max}$ (KBr) 1755, 1662, 1590, 1528 and 1382Cm$^{-1}$; $\delta_H$ (250MHz. $(CD_3)_2SO$) 0.56 (4H, m), 2.71 (1H, m), 2.74 and 2.93 (2H, ABq, J 16Hz), 3.84 (3H, s), 4.94 (1H, d, J 4.5Hz), 5.46 (1H, dd, J 4.5, 7.1Hz), 6.78 (1H, s), 7.27 (2H, s, exch.), 9.49 (1H, d, J 7.1Hz, exch.); [Mass spectrum: +ve ion (thioglycerol) $MH^+$ (446) and $MNa^+$ (468)].

Example 4

Pivaloyloxymethyl (6R,7R)-7-[2-(2-Aminothiazol-4-yl)-2-(Z)-methoxyiminoacetamidol-3-cyclopropylceph-3-em-4-carboxylate

Bromomethyl pivaloate (0.09g) in acetone (2ml) was stirred at room temperature with dry sodium iodide for 30min. The acetone was removed in vacuo and the crude iodide treated with a solution of sodium (6R,7R)-7-[2-(2-aminothiazol-4-yl)-2-(Z)-methoxyiminoacetamido]-3-cyclopropylceph-3-em-4-carboxylate (0.17g) in DMF (1ml), at room temperature for 30min. The reaction mixture was diluted with ethyl acetate (20ml) and washed with water and brine. After drying and removal of solvent the crude product was flash chromatographed on silica gel eluting with ethyl acetate. The title compound was obtained as an off-white solid (0.128g, 60%); $\nu_{max}$ ($CH_2Cl_2$) 3650-3200 (v br), 1750 (v br), 1680, 1605 and 1530cm$^{-1}$; $\delta_H$(250MHz, $CDCl_3$) 0.71 (1H, m), 0.95 (1H, m), 1.24 (9H, s), 2.62 (1H, m), 2.95 (2H, s), 4.06 (3H, s), 5.10 (1H, d, J 4.6Hz), 5.51 (1H, s, exch.), 5.88 and 5.93 (2H, ABq, J 5.5Hz), 5.94 (1H, dd, J 4.6, 8.8Hz), 6.79 (1H, s), 7.90 (1H, d, J 8.8Hz, exch.); [Mass spectrum: +ve ion (thioglycerol) $MH^+$ (538)].

Example 5

(6R,7R)-7-[(2R)-2-Amino-2-(4-hydroxyphenyl)acetamido]-3-cyclopropylceph-3-em-4-carboxylic acid

a) Diphenylmethyl (6R,7R)-7-[(R)2-t-butoxycarbonylamino-2-(4-hydroxyphenyl)acetamido]-3-cyclopropylceph-3-em-4-carboxylate

Diphenylmethyl (6R,7R)-7-amino-3-cyclopropylceph-3-em-4-carboxylate (0.36g) in dry tetrahydrofuran (THF, 10ml), cooled to 0-5°C in ice/water was treated with dicyclohexylcarbodiimide (0.274g). A solution of (R)2-t-butoxycarbonylamino-2-(4-hydroxyphenyl)acetic acid (0.355g) in THF (3ml) was added dropwise. The reaction mixture was left at 0-5°C for 30min then at room temperature for a further 30min. The solution was filtered through Celite and the solvent removed in vacuo. The crude product was purified by flash chromatography on silica gel eluting with 50% ethyl acetate/hexane. After trituration with ethyl acetate/ether the title compound was obtained as a white solid (0.511g, 88%); $\nu_{max}$ ($CH_2Cl_2$) 3550 (v.w.), 3400 (w), 1780, 1710, 1690 (shoulder), 1510 and 1490cm$^{-1}$; $\delta_H$ (250MHz, $CDCl_3$) 0.50 (1H, m), 0.76 (1H, m), 1.43 (9H, s), 2.60 (1H, m), 2.60 and 2.73 (2H, ABq, J 16Hz), 4.94 (1H, d, J 4.4Hz), 5.13 (1H, br d, J 5.1Hz), 5.62 (1H, dd, J 4.4, 8.8Hz), 5.69 (1H, br d, J 5.1Hz), 6.66 and 7.13 (4H, ABq, J 8.4Hz), 6.93 (1H, s), 6.94 (1H, d, J 8.8Hz, exch.), 7.25-7.43 (10H, m); [Mass spectrum: +ve ion (3-nitrobenzylalcohol) $MH^+$ (656), $MNa^+$ (678)].

b) (6R,7R)-7-[(2R)2-Amino-2-(4-hydroxyphenyl)acetamidol-3-cyclopropylceph-3-em-4-carboxylic acid

Diphenylmethyl (6R,7R)-7-[(2R)2-t-butoxycarbonylamino-2-(4-hydroxyphenyl)acetamido]-3-cyclopropylceph-3-em-4- carboxylate (0.45g) in dichloromethane (15ml) was cooled to 0-5°C in ice/water and treated with anisole (4ml) and trifluoroacetic acid (TFA, 5ml). The reaction mixture was warmed to room temperature for 2h. The solvent and excess reagents were removed in vacuo. The residue was dissolved in saturated sodium bicarbonate and extracted with ethyl acetate. The pH was adjusted to ca. 8 and the solution concentrated. The crude product was chromatographed on HP20SS eluting with 0-6% THF/water. The fractions containing the product were combined, concentrated and freeze-dried to give the title compound as a white foam (0.169g, 63%); $\nu_{max}$ (KBr) 1751, 1682, 1609, 1560, 1515 and 1456cm$^{-1}$; $\delta_H$ (250MHz, $D_2O$ + DCl) 0.52 (1H, m), 0.63 (1H, m), 0.76 (2H, m), 2.36 (1H, m), 2.62 and 2.75 (2H, ABq, J 16Hz), 4.95 (1H, d, J 4.3Hz), 5.04 (1H, s), 5.37 (1H, d, J 4.3Hz), 6.81 (2H, m), 7.24 (2H, m); Mass spectrum: +ve ion (thioglycerol) $MH^+$ (390), $MNa^+$ (412), $2MH^+$ (779) and $2MNa^+$ (801)].

Example 6

Disodium (6R,7R)-7-[2-(2-Aminothiazol-4-yl)-2-(Z)-carboxymethoxyiminoacetamido]-3-cyclopropyceph-3-em-4-carboxylate

a) t-Butyl (6R,7R)-7-[2-(Z)-t-Butoxycarbonylmethoxyimino-2-(2-tritylaminothiazol-4-yl)acetamido]-3-cyclopropylceph-3-em-4-carboxylate

Oxalyl chloride (44μL, 0.5mmol) then 2-(Z)-t-butoxycarbonylmethoxyimino-2-(2-tritylaminothiazol-4-yl)acetic acid (0.226g, 0.5mmol) in dichloromethane (2ml) and then t-butyl (6R,7R)-7-amino-3-cyclopropylceph-3-em-4-carboxylate (0.118g, 0.4mmol) and pyridine (81μL, 1mmol) in dichloromethane (2ml) were added at 10min intervals to DMF (40μL, 0.5mmol) in dichloromethane (2ml) cooled to -10±5°C. The mixture was stirred for 1h without further cooling, washed with very dilute hydrochloric acid, dried, concentrated and flash chromatographed on silica gel, eluting with 40% ethyl acetate in hexane (0.212g, 65%); $\nu_{max}$ ($CH_2Cl_2$) 3400, 1785, 1728, 1685, 1525, 1365 and 1155cm$^{-1}$; $\delta_H$ (250MHz, $CDCl_3$) 0.4-1.0 (4H, m), 1.43 (9H, s), 1.53 (9H, s), 2.4-2.55 (1H, m), 2.84 and 2.96 (2H, ABq, J 17.1Hz), 4.75 (2H, s), 5.02 (1H, d, J 4.6Hz), 5.77 (1H, dd, J 8.5, 4.6Hz), 6.82 (1H, s), 6.99 (1H, s), 7.30 (15H, s), 8.54 (1H, d, J 8.4Hz); [Mass spectrum: +ve ion (thioglycerol) $MH^+$ (822)].

b) Disodium (6R,7R)-7-[2-(2-Aminothiazol-4-yl)-2-(Z)-carboxymethoxyiminoacetamido]-3-cyclopropylceph-3-em-4-carboxylate

TFA (2ml) was added to t-butyl (6R,7R)-7-[2-(Z)-t-butoxycarbonylmethoxyimino-2-(2-tritylaminothiazol 4-yl)acetamido]-3-cyclopropylceph-3-em-4-carboxylate (0.252g) in anisole (0.5ml) and stirred for 3.5h. The mixture was concentrated, diluted with ethyl acetate (10ml) and water (11ml), adjusted to pH 7 (saturated aqueous sodium bicarbonate) and the aqueous layer reduced in volume and chromatographed on HP20SS eluting with water. Fractions containing the product were bulked and freeze-dried (81mg, 52%); $\lambda_{max}$ ($H_2O$) 232 (ε 12,038), 256nm (ε 10,974); $\nu_{max}$ (KBr) 1756, 1675, 1604, 1383 and 1067cm$^{-1}$; $\delta_H$ (250MHz, $(CD_3)_2SO$ + $D_2O$) 0.5-0.65 (4H, m), 2.65-2.85 (3H, m), 4.22 (2H, s), 4.91 (1H, d, J 4.4H), 5.43 (1H, d, J 4.4Hz), 6.84 (1H, s); [Mass spectrum: +ve ion (thioglycerol) $MH^+$ (512), $MNa^+$ (534)].

Example 7

Sodium (6R,7R)-7-[2-(2-Aminothiazol-4-yl)-2-(Z)-hydroxyiminoacetamido]-3-cyclopropylceph-3-em-4-carboxylate

a) t-Butyl (6R.7R)-3-Cyclopropyl-7-[2-(2-tritylamino thiazol-4-yl)-2-(Z)-trityloxyiminoacetamido]ceph-3-em-4-carboxylate

Methanesulphonyl chloride (34μL, 0.44mmol) was added to sodium 2-(2-tritylaminothiazol-4-yl)-2-(Z)-trityloxyiminoacetate (0.277g, 0.4mmol) in DMF (2ml) at -45°C. The mixture was stirred and allowed to warm to -15°C over 0.5h then t-butyl (6R,7R)-7-amino-3-cyclopropylceph-3-em-4-carboxylate (0.118g, 0.4mmol) and pyridine (35μL, 0.44mmol) in DMF (1ml) were added. The reaction mixture was stirred 1h without further cooling, diluted with ethyl acetate (30ml), washed with water (2 x 20ml) and brine (20ml), dried, concentrated and flash chromatographed on silica gel eluting with 50% ethyl acetate in hexane to give the title compound (0.280g, 74%); $\nu_{max}$ ($CH_2Cl_2$) 3400, 1785, 1715, 1690 and 1155cm$^{-1}$ $\delta_H$ (90MHz, $CDCl_3$) 0.4-1.0 (4H, m), 1.53 (9H, s), 2.1-2.4 (1H, m), 2.57 and 2.87 (2H, ABq, J 17.1Hz), 4.95 (1H, d, J 5Hz), 5.88 (1H, dd, J 8, 5Hz), 6.41 (1H, s), 6.75 (1H, br s), 7.23 (30H, s); [Mass spectrum: +ve ion (thioglycerol, 3-nitrobenzylalcohol, sodium acetate) $MNa^+$ (972)].

b) Sodium (6R,7R)-7-[2-(2-Aminothiazol-4-yl)-2-(Z)-hydroxyiminoacetamido]-3-cyclopropylceph-3-em-4-carboxylate

t-Butyl (6R,7R)-3-cyclopropyl-7-[2-(2-tritylaminothiazol-4-yl)-2-(Z)-trityloxyiminoacetamido]ceph-3-em-4-carboxylate (0.280g) in TFA (2ml) was stirred for 10min then evaporated to dryness. Toluene (2ml) was added then re-evaporated. The residue in aqueous THF was adjusted to pH 5 (0.1M NaOH), washed with ether (2 x 10ml) concentrated and chromatographed on HP20SS eluting with 0-6% THF in water. Fractions containing the title compound were combined and freeze-dried (28mg, 22%), $\lambda_{max}$ ($H_2O$) 221 (ε 16,536), 262nm (ε 12,734); $\nu_{max}$ (KBr) 1751, 1660, 1618, 1382 and 1046cm$^{-1}$; $\delta_H$ (250MHz, $D_2O$) 0.3-0.9 (4H, m), 1.75-1.95 (1H, m), 2.97 and 3.29 (2H, ABq, J 17.4Hz), 5.11 (1H, d, J 4.5Hz), 5.69 (1H, d, J 4.5Hz), 6.95 (1H, s); [Mass spectrum: +ve ion (thioglycerol) $MH^+$ (432), $MNa^+$ (454)].

Example 8

Sodium (6R,7R)-7-[2-(2-Aminothiazol-4-yl)-2-(Z)-methoxyiminoacetamido]-3-[(1RS,2RS)-1-methoxycarbonylcycloprop-2-yl]ceph-3-em-4-carboxylate

a) (3R,4R)-4-[(1RS,2RS)-1-Methoxycarbonylcycloprop-2-ylcarbonylmethylthio]-3-phenoxyacetamidoazetidin-2-one

Methyl (1RS,2RS)-2-acetylcyclopropylcarboxylate, prepared using the method described for the ethyl ester

(G.B. Payne , J.Org.Chem., 1967, 32, 3351), was converted to methyl (1RS,2RS)-2-(bromoacetyl)cyclopropylcarboxylate and this used to alkylate ( 3R,4R)-4-mercapto-3-phenoxyacetamidoazetidin-2-one as described in Example 1a to provide the title compound (80%); (Found: $M^+$, 392.1048. $C_{18}H_{20}N_2O_6S$ requires M, 392.1042); $\nu_{max}$ ($CH_2Cl_2$) 3395, 1780, 1725, 1690, 1325, 1205 and 1170cm$^{-1}$; $\delta_H$ (90Hz, $CDCl_3$) 1.3-1.6 (2H, m), 2.05-2.35 (1H, m), 2.4-2.7 (1H, m), 3.46 (2H, s), 3.65 3H, s), 4.53 (2H, s), 4.92 (1H, d, J 5Hz) 5.51 (1H, dd, J 9,5Hz), 6.8-7.4 (6H, m), 7.75 (1H, d, J 9Hz).

b) t-Butyl (2RS)-2-Hydroxy-2-[(3R,4R)-4-[(1RS,2RS)-1-methoxycarbonylcycloprop-2-yl-carbonylmethylthio]-3-phenoxyacetamidcazetidin-2-on-1-yl]acetate

Prepared according to the method described in Example 1b (94%); $\nu_{max}$ ($CH_2Cl_2$) 3460, 3400, 1780, 1730, 1690 and 1150cm$^{-1}$ [Mass spectrum: +ve ion (3-nitrobenzylalcohol, sodium acetate) $MNa^+$ (545)].

c) t-Butyl 2-[(3R,4R)-4-[(1RS,2RS)-1-Methoxycarbonylcycloprop-2-ylcarbonylmethylthio]-3-phenoxyacetamidoazetidin-2-on-1-yl]-2-tri-n-butylphosphoranylideneacetate

Thionyl chloride (0.47ml, 6.4mmol) in THF (5ml) was added dropwise to t-butyl (2RS)-2-hydroxy-2-[(3R, 4R)-4-[1RS,2RS)-1-methoxycarbonylcycloprop-2-ylcarbonylmethylthio]-3-phenoxyacetamidoazetidin-2-on-1-yl]acetate (2.328g, 4.3mmol) and 2,6-lutidine (0.75ml, 6.4mmol) in THF (20ml) at $<-20°C$. The mixture was stirred 0.5h without further cooling, filtered and the filtrate evaporated, toluene added to the residue and re-evaporated to provide crude t-butyl (2RS,-2-chloro-2-[(3R,4R)-4-[(1RS, 2RS)-1-methoxycarbonylcycloprop-2-ylcarbonylmethylthio]-3-phenoxyacetamidoazetidin-2-on-1-yl]acetate as a foam. To this in 1,4-dioxan (10ml) was added tri-n-butylphosphine (2.35ml, 9.4mmol), stirred 0.25h, diluted with ethyl acetate then washed with dilute sodium bicarbonate, water and brine, dried, concentrated and flash chromatographed on silica gel eluting with 40-75% ethyl acetate in hexane to give the title compound (1.430g, 47%); $\nu_{max}$ ($CH_2Cl_2$) 3405, 1760, 1725, 1685 and 1170cm$^{-1}$. [Mass spectrum: +ve ion (3-nitrobenzylalcohol, sodum acetate) $MNa^+$ 729)].

d) t-Butyl (6R,7R)-3-[(1RS,2RS)-1-Methoxycarbonylcycloprop-2-yl]-7-phenoxyacetamidoceph-3-em-4-carboxylate

The phosphorane (1.430g) and benzoic acid (20mg) in toluene (30ml) were purged with argon and heated at 120°C for 3h, cooled and flash chromatographed on silica gel eluting with 35-40% ethyl acetate in hexane to provide the title compound (0.700g, 71%); $\nu_{max}$ ($CH_2Cl_2$) 3400, 1782. 1722, 1695, 1510 and 1150cm$^{-1}$; $\delta_H$ (250MHz, $CDCl_3$) 1.0-1.9 (3H, m), 1.55 (9H, s), 2.6-2.7, 2.75-2.85 (together 1H, 2m), 2.91 and 3.27. 2.99 and 3.23 (together 2H, 2ABq, J 17.9, 17.8Hz), 3.70, 3.72 (together 3H, 2s), 4.57 (2H,s), 4.97 (1H, d, J 4.7Hz). 5.86 (1H, m), 6.9-7.4 (6H, m). [Mass spectrum: +ve ion (3-nitrobenzylalcohol, sodium acetate) $MNa^+$ (511)].

e) t-Butyl (6R,7R)-7-Amino-3-[(1RS,2RS)-1-methoxycarbonylcycloprop-2-yl]ceph-3-em-4-carboxylate

Phosphorus pentachloride (0.441g, 2.1mmol) in dichloromethane (11ml) was added to t-butyl (6R,7R)-3-[(1RS, 2RS)-1-methoxycarbonylcycloprop-2-yl]-7-phenoxyacetamidoceph-3-em-4-carboxylate (0.700g,1.4mmol) and N-methylmorpholine (308µl, 2.8mmol) in dichloromethane (15ml) at -20°C. Stirred at -15±5°C for 0.5h, methanol (5ml) added and stirred 0.75h, water (10ml) added and stirred vigorously for 1h. The dichloromethane was removed in vacuo, the aqueous residue washed with ether then adjusted to pH6 with dilute ammonium hydroxide in the presence of ethyl acetate. The organic phase was washed with brine, dried, concentrated and flash chromatographed on silica gel eluting with 40-60% ethyl acetate in hexane to give the title compound (0.203g, 41%); (Found $M^+$, 354.1248. $C_{16}H_{22}N_2O_5S$ requires M, 354.1249); $\nu_{max}$ ($CH_2Cl_2$) 1778, 1725, 1365, 1175 and 1150cm$^{-1}$; $\delta_H$ (250MHz,$CDCl_3$) 1.0-1.9 (5H, m), 1.55 (9H, s), 2.55-2.65, 2.75-2.85 (together 1H, 2m), 2.91 and 3.27, 3.01 and 3.21 (together 2H, 2ABq, J 17.9, 17.8Hz), 3.69, 3.71 (together 3H, 2s), 4.71 (1H, d, J 5.0Hz), 4.89 (1H, d, J 5.0Hz).

f) t-Butyl (6R,7R)-3-[(1RS,2RS)-1-Methoxycarbonylcyclprop-2-yl]-7-[2-(Z)-methoxyimino-2-(2-tritylaminothiazol-4-yl)acetamido]ceph-3-em-4-carboxylate

Methanesulphonyl chloride (39µl1, 0.5mmol) was added to 2-(2-tritylaminothiazol-4-yl)-2-(Z)-methoxyiminoacetic acid hydrochloride (192mg, 0.4mmol) and diisopropylethylamine (139µl, 0.8mmol) in DMF (1ml) at -40°C. The mixture was stirred 0.5h at -30±10°C then t-butyl (6R,7R)-7-amino-3-[(1RS,2RS)-1-methoxycarbonylcycloprop-2-yl]ceph-3-em-4-carboxylate (89mg, 0.25mmol) in DMF (2ml) and pyridine (41µl, 0.5mmol) were added. The mixture was stirred 1h, diluted with ethyl acetate, washed with water (2x20ml) and brine 20ml), dried, concentrated and flash chromatographed on silica gel eluting with 40-50% ethyl acetate in hexane to give the title compound (0.150g, 77%); $\nu_{max}$ ($CH_2Cl_2$) 3395, 1780, 1725, 1683, 1520, 1370, 1238 and 1040cm$^{-1}$; $\delta_H$ (250MHz, $CDCl_3$) 1.0-1.9 (3H, m), 1.53 (9H, s) 2.6-2.7 and 2.75-2.85 (together 1H, 2m), 2.90 and 3.26, 2.98 and 3.24 (together 2H, 2ABq, J 18.0, 17.9Hz), 3.69, 3.72 (together 3H, 2s) 4.07 (3H, s), 4.99 (1H, d, J4.8Hz), 5.87 (1H, d, J 8.8, 4.8Hz), 6.7-6.8 (2H, m), 7.02 (1H, s), 7.2-7.4 (15H, m). [Mass spectrum: +ve ion (3-nitrobenzyl alcohol, sodium acetate) $MNa^+$ (802)].

g) Sodium (6R,7R)-7-|2-(2-Aminothiazol4-yl)-2-(Z)-methoxyiminoacetamidol-3-[(1RS,2RS)-1-methoxycarbonylcycloprop-2-yl]ceph-3-em-4-carboxylate

t-Butyl (6R,7R)-3-[(1RS,2RS)-1-methoxycarbonylcycloprop-2-yl]-7-[2-(Z)-methoxyimino-2-(2-tritylaminothiazol-4-yl)acetamido]ceph-3-em-4-carboxylate (0.150g, 0.2mmol) was dissolved in 0.1M HCl in 90% formic acid (2ml) and set aside for 0.75h. Conc. hydrochloric acid (50µl) was added, the mixture set aside for a further 0.75h then evaporated to dryness, water added and adjusted to pH6.5 (0.25M NaOH) then chromatographed on HP20SS eluting with 0-3% THF in water. Fractions containing the title compound were combined. concentrated in vacuo and freeze dried (64mg, 64%); $\nu_{max}$ (KBr) 1761, 1711, 1670, 1601, 1532, 1361 and 1039cm$^{-1}$; $\delta_H$ (250MHz, $D_2O$) 1.1-1.4 (2H, m), 1.75-1.95 (1H, m), 2.25-2.4, 2.4-2.5 (together 1H, m), 3.08 and 3.45, 3.10 and 3.41 (together 2H, 2ABq, J 17.7, 17.7Hz) 3.66, 3.67 (together 3H, 2s), 3.94 (1H, s), 5.11 (1H, d, J 4.6Hz), 5.72 5.73 (together 1H, 2d, J 4.6Hz), 6.98 (1H, s). [Mass spectrum: +ve ion (thioglycerol) $MH^+$ (504)].

Example 9

Sodium (6R,7R)-7-[2-(2-Aminothiazol-4-yl)-2-(Z)-methoxyiminoacetamido]-3-(1-methoxycarbonylcycloprop-1-yl)ceph-3-em-4-carboxylate

a) Methyl 1-Acetylcyclopropylcarboxylate

Methyl acetoacetate (8.6ml, 80mmol), 1,2-dibromoethane (7ml, 80mmol) and potassium hydroxide (11.2g, 200mmol) in DMSO (80ml) were stirred for 48h, poured into water, acidified and extracted with ether (3x50ml). The extracts were washed with water (2x100ml) and brine 50ml), dried, concentrated and flash chromatographed on silica gel eluting with 5-10% ethyl acetate in hexane to yield the title compound (4.18g, 37%); $\delta_H$ (90MHz, $CDCl_3$) 1.46 (4H,s), 2.44 (3H,s), 3.72 (3H,s).

b) (3R,4R)-4-(1-Methoxycarbonylcycloprop-1-yl)-3-phenoxyacetamidoazetidin-2-one

Prepared using the method described in Example 1a (90%), recrystallised from ethyl acetate-hexane, mp 121°C; (Found: C, 55.05; H, 4.93; N, 7.15; S, 7.83%. $C_{18}H_{20}N_2O_6S$ requires: C, 55.09; H, 5.14; N, 7.14; S, 8.17%); $\delta_H$ (250MHz, DMSO-$d_6$) 1.4-1.55 (4H,m), 3.66 (3H,s), 3.86 (2H,s), 4.56 and 4.62 (2H, ABq, J 5.0Hz) 4.88 (1H, d, J 4.5Hz), 5.25 (1H, dd, J 8.4, 4.4Hz), 6.9-7.4 (5H, m), 8.81 (1H, s), 8.98 (1H, d, J 8.6Hz).

c) t-Butyl (2RS)-2-Hydroxy-2-[(3R,4R)-4-(1-methoxycarbonylcycloprop-1-ylcarbonylmethylthio)-3-phenoxyacetamidoazetidin-2-on-1-yl]acetate

Prepared according to the methods described in Example 1b (80%).

d) t-Butyl (2RS)-2-[(3R,4R)-4-(1-Methoxycarbonylcycloprop-1-ylcarbonylmethylthio)-3-phenoxyacetamidoazetidin-2-on-1-yl]-2-tri-n-butylphosphoranylideneacetate

Prepared by the method described in Example 8c (55%); $\nu_{max}$ ($CH_2Cl_2$) 3405, 1762, 1730, 1690, 1240 and 1170cm$^{-1}$. [Mass spectrum: +ve ion (3-nitrobenzylalcohol, sodium acetate) $MNa^+$ (729)].

e) t-Butyl (6R,7R)-3-(1-Methoxycarbonylcycloprop-1-yl)-7-phenoxyacetamidoceph-3-em-4-carboxylate

The phosphorane was heated in toluene for 48h as described in Example 8d to provide the title compound (2%); (Found: $M^+$, 488.1606. $C_{24}H_{28}N_2O_7S$ requires M, 488.1617); $\nu_{max}$ ($CH_2Cl_2$) 3400, 1782, 1720, 1691, 1225 and 1150cm$^{-1}$; $\delta_H$ (90MHz, $CDCl_3$) 1.3-1.7 (13H, m), 3.42 (2H, s), 3.67 (3H, s), 4.52 (2H, s), 4.92 (1H, d, J 5Hz), 5.86 (1H, dd, J 9, 5Hz), 6.8-7.5 (6H, m).

f) t-Butyl(6R,7R)-7-Amino-3-(1-methoxycarbonylcycloprop-1-yl)ceph-3-em-4-carboxylate

Prepared by the method described in Example 8c (25%); (Found: $M^+$, 354.1258. $C_{16}H_{22}N_2O_5S$ requires M, 354.1249); $\nu_{max}$ ($CH_2Cl_2$) 1775, 1722, 1365 and 1150cm$^{-1}$.

g) t-Butyl (6R,7R)-3-(1-Methoxycarbonylcyclopropo-1-yl)-7-[2-(Z)-methoxyimino-2-(2-tritylaminothiazol-4-yl)acetamido]ceph-3-em-4-carboxylate

Prepared by the method described in Example 8f (36%); $\nu_{max}$ ($CH_2Cl_2$) 3398, 1781, 1721, 1685, 1520, 1365, 1150 and 1040cm$^{-1}$; $\delta_H$ (250MHz, $CDCl_3$) 1.26 (4H, s), 1.52 9H, s), 3.43 and 3.48 (2H, ABq, J 18.1Hz), 3.70 (3H, s), 4.09 (3H, s), 4.96 (1H, d, J 4.9Hz), 5.92 (1H, dd, J 9.0, 4.9Hz), 6.7-6.9 (2H, m) 7.11 (1H, s), 7.2-7.4 (15H, m). [Mass spectrum: +ve ion (3-nitrobenzyl alcohol, sodium acetate) $MNa^+$ (802)].

h) Sodium (6R,7R)-7-[2-(2-Aminothiazol-4-yl)-2-(Z)-methoxyiminoacetamido]-3-(1-methoxycarbonylcycloprop-1-yl)ceph-3-em-4-carboxylate

Prepared by the method described in Example 8g (62%); $\nu_{max}$ (KBr) 1764, 1710, 1671, 1604, 1532, 1375, 1158

and $1040cm^{-1}$; $\delta_H$ (250MHz, $D_2O$) 1.0-1.6 (4H, m), 3.48 and 3.59, (2H, ABq, J 18.OHz), 3.64 (3H, s), 3.96 (3H, s), 5.08 (1H, d, J 4.7Hz), 5.76 (1H, d, J 4.6Hz), 6.99 (1H, s). [Mass spectrum: +ve ion (thioglycerol) $MH^+$ (504)].

Example 10

Sodium(6R,7R)-7-[2-Aminothiazol-4-yl)-2-(Z)-methoxyiminoacetamido]-3-[(2RS)-1,1-bismethoxycarbonylcycloprop-2-yl]ceph-3-em-4-carboxylate

a) Diphenylmethyl(6R,7R)-3-(2,2-Bismethoxycarbonylvinyl)-7-phenylacetamidoceph-3-em-4-carboxylate

Dimethyl ketomalonate (0.637g) in dichloromethane (5ml) was added to a suspension of diphenylmethyl (6R,7R)-7-phenylacetamido-3-triphenylphosphoranylideneethyl-ceph-3-em-4-carboxylate (1.65g) in dichloromethane (20ml). After 0.75h the mixture was washed with dil. hydrochloric acid and brine, dried, concentrated and flash chromatographed on silic gel eluting with 40% ethyl acetate-hexane to give the title compound (0.637g, 47%); $\nu_{max}$ ($CH_2Cl_2$) 3400, 1785, 1725, 1682, 1490, 1368 and $1215cm^{-1}$; $\delta_H$ (250MHz, $CDCl_3$) 3.35 and 3.53 (2H, ABq, J 18.0Hz), 3.62 and 3.68 (2H, ABq, J 16.1Hz), 3.72 (3H, s), 3.76 (3H, s), 4.98 (1H, d, J5.1Hz), 5.90 (1H, dd, J 9.0, 5.0Hz), 6.06 (1H, d, J 9.1Hz), 6.94 (1H, s), 7.2 - 7.5 (2H, m), 7.69 (1H, s). [Mass spectrum: +ve ion (3-nitrobenzyl alcohol, sodium acetate) $MNa^+$ (649)].

b) Diphenylmethyl (6R,7R)-3-[(4RS)-3,3-Bismethoxycarbonylpyrrazolin-4-yl)-7-phenylacetamidoceph-3-em-4-carboxylate

Excess etheral diazomethane was added to diphenylmethyl (6R,7R)-3-(2,2-bismethoxycarbonylvinyl)-7-phenylacetamidoceph-3-em-4-carboxylate (0.630g) in dichloromethane (10ml). After 2h, the mixture was concentrated in vacuo and flash chromatographed to provide the title compound (0.407g, 61%); $\delta_H$ (250MHz, $CDCl_3$) 2.48 and 2.87, 2.52 and 2.76 (together 2H, 2ABq, J 17.0, 17.7Hz), 3.5-3.8 (8H, m), 4.45-4.65 (1H, m), 4.8-5.1 (3H, m), 5.72. S.75 (together 1H, 2dd, J 8.6, 4.7Hz) 6.06, 6.10 (together 1H, 2d, J 8.7Hz), 6.95, 6.99 (together 1H, 2s), 7.2 - 7.45 (15H, m).

c) Diphenylmethyl (6R,7R)-3-[(2RS)-1,1-Bismethoxycarbonylcycloprop-2-yl)-7-phenylacetamidoceph-3-em-4-carboxylate

The pyrrazoline (400mg) in ethyl acetate (10ml) was heated under reflux for 2 days then concentrated and flash chromatographed to give the title compound 174mg, 45%). [Mass spectrum: +ve ion (3-nitrobenzyl alcohol, sodium acetate) $MNa^+$ (663)].

d) Diphenylmethyl (6R,7R)-7-Amino-3-[(2RS)-1,1-bismethoxycarbonylcycloprop-2-yl]ceph-3-em-4-carboxylate

Prepared by the method described in Example 8e (49%). [Mass spectrum: +ve ion (3-nitrobenzyl alcohol, sodium acetate) $MNa^+$ (545)].

e) Diphenylmethyl (6R,7R)-3-[(2RS)-1,1-Bismethoxycarbonylcycloprop-2-yl]-7-[2-(Z)-methoxyimino-2-(2-tritylaminothiazol-4-yl)acetamido]ceph-3-em-4-carboxylate

Prepared by the method described in Example 8f (63%). [Mass spectrum: +ve ion (3-nitrobenzyl alcohol, sodium acetate) $MNa^+$ (970)].

f) Sodium (6R,7R)-7-[2-(2-Aminothiazol-4-yl)-2-(Z)-methoxyiminoacetamido]-3-[(2RS)-1,1-bismethoxycarbonylcycloprop-2-yl]ceph-3-em-4-carboxylate

Prepared using the method described in Example 8g (49%); $\nu_{max}$ (KBr) 1762, 1724, 1670, 1610, 1533, 1380, 1257 and $1037cm^{-1}$; $\delta_H$ (250MHz, $D_2O$) 2.16 (2H, s), 3.55-3.9 (8H, m), 3.96 (3H, s), 5.22 (1H, d, J 4.7Hz), 5.80 (1H, d, J 4.7Hz), 7.00 (1H, s). [Mass spectrum: +ve ion (thioglycerol) $MH^+$ (562)].

In Vitro Biological Data

MIC (μg/ml)

| Example No. | Organism | |
|---|---|---|
| | E.coli NCTC 10418 | S. aureus Oxford |
| 3 | 0.25 | 4 |
| 5 | 32 | 0.5 |
| 6 | 1 | 64 |
| 7 | 4 | 0.5 |
| 8 | 0.5 | 8 |
| 9 | 16 | - |
| 10 | 1 | 64 |

## Claims

1. A compound of formula (I) or a salt thereof:

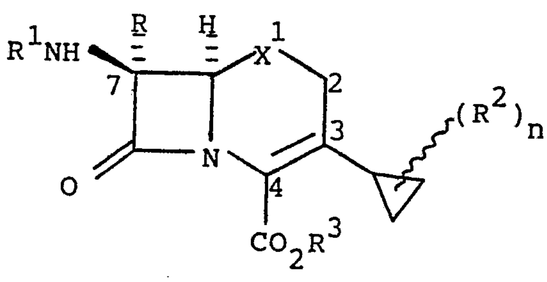

whereinR is hydrogen, methoxy or formamido;
$R^1$ is an acyl group, in particular that of an antibacterially active cephalosporin;
$R^2$ is optionally substituted alkyl, acyl, alkoxycarbonyl, halogen, aryl, heterocyclyl, carboxy, carboxamido, or cyano;
$CO_2R^3$ is carboxy or a carboxylate anion, or $R^3$ is a readily removable carboxy protecting group; X is S,SO,$SO_2$,O or $CH_2$; and n is zero or an integer from 1 to 5.

2. A compound as claimed in claim 1 in which $R^1$ is an acyl group of formula (a) to (f):

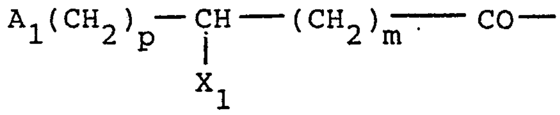

(a)

$A_2CO—$ (b)

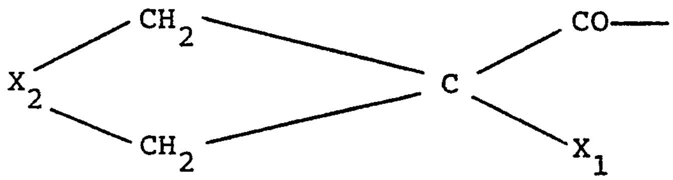

(c)

$A_2—X_3—(CH_2)_p—CO—$ (d)

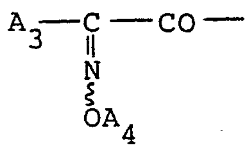

(e)

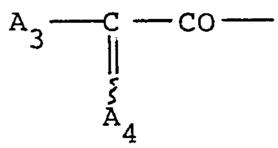

(f)

wherein p is 0, 1 or 2; m is 0, 1 or 2; $A_1$ is $C_{1-6}$ alkyl, substituted $C_{1-6}$ alkyl, $C_{3-6}$ cycloalkyl, cyclohexenyl, cyclohexadienyl, an aromatic (including heteroaromatic) group, such as phenyl, substituted phenyl, thienyl, pyridyl, or an optionally substituted thiazolyl group, a $C_{1-6}$ alkylthio group or $C_{1-6}$ alkyloxy; $X_1$ is a hydrogen or halogen atom, a carboxylic acid, carboxylic ester, sulphonic acid, azido, tetrazolyl, hydroxy, acyloxy, amino, ureido, acylamino, heterocyclylamino, guanidino or acylureido group; $A_2$ is an aromatic group, for example a phenyl, 2,6-dimethoxyphenyl,2-alkoxy-1-naphthyl, 3-arylisoxazolyl, or a 3-aryl-5-methylisoxazolyl group, such as 3-(2-chloro-6-fluorophenyl)5-methylisoxazol-4-yl; a substituted alkyl group; or a substituted dithietane; $X_2$is a $CH_2OCH_2$, $CH_2SCH_2$ or alkylene group; $X_3$ is an oxygen or sulphur atom; $A_3$ is an aryl or heteroaryl group such as phenyl, substituted phenyl or aminothiazolyl in which the amino group is optionally protected; and $A_4$ is hydrogen, $C_{1-6}$ alkyl, $C_{3-6}$ cycloalkyl, $C_{1-6}$ alkoxycarbonyl($C_{1-6}$)alkyl, $C_{2-6}$ alkenyl, carboxy($C_{1-6}$)alkyl, $C_{2-6}$ alkynyl, aryl and aryl($C_{1-6}$)alkyl.

3. A compound as claimed in claim 2 in which $R^1$ is an acyl group of formula (a) wherein $R_1$ is phenyl or 4-hydroxyphenyl, $X_1$ is amino or t-butoxycarbonylamino and p and m are 0; or R is an acyl group of formula (d) wherein $A_2$ is phenyl, $X_3$ is oxygen and p is 0; or $R^1$ is an acyl group of formula (e) wherein $A_3$ is 2-aminothiazol-4-yl, 2-tritylaminothiazol-4-yl or 2-chloroacetamidothiazol-4-yl and $A_4$ is hydrogen, methyl, carboxymethyl, t-butoxycarbonylmethyl or triphenylmethyl.

4. A compound as claimed in any one of claims 1 to 3 in which R is hydrogen; X is sulphur; n is zero, or n is 1 or 2 and $R^2$ is methoxycarbonyl; and $CO_2R^3$ is carboxy or a carboxylate anion, or $R^3$ is t-butyl, diphenylmethyl or pivaloylmethyl.

5. A compound of formula (Ia) or a pharmaceutically acceptable salt or pharmaceutically acceptable *in vivo* hydrolysable ester thereof:

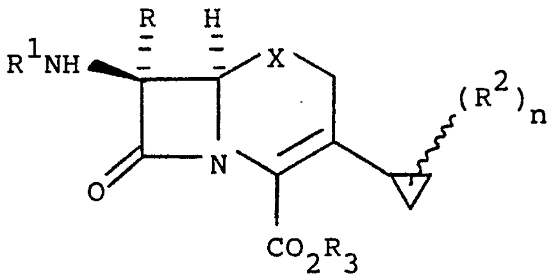

(Ia)

wherein R,$R^1$,$R^2$, X and n are as defined with respect to formula (I) in any one of claims 1 to 4 and $CO_2R_3$ is carboxy or a carboxylate anion.

6. A compound of formula (Ia) as claimed in claim 5, or a pharmaceutically acceptable salt or pharmaceutically acceptable in vivo hydrolysable ester thereof, selected from:

(6R,7R)-7-[2-(2-aminothiazol-4-yl)-2-(Z)-methoxyiminoacetamido]-3-cyclopropylceph-3-em-4-carboxylic acid,

(6R,7R)-7-[(2R)-2-amino-2-(4-hydroxyphenyl)acetamido]-3-cyclopropylceph-3-em-4-carboxylic acid,

(6R,7R)-7-[2-(2-aminothiazol-4-yl)-2-(Z)-carboxymethoxyiminoacetamido]-3-cyclopropylceph-3-em-4-carboxylic acid,

(6R,7R)-7-[2-(2-aminothiazol-4-yl)2-(Z)-hydroxyiminoacetamido]3-cyclopropylceph-3-em-4-carboxylic acid,

( 6R,7R)-7-[2-(2-aminothiazol-4-yl)-2-(Z)-methoxyiminoacetamido]-3-[1RS,2RS)-1-methoxycarbonylcycloprop-2-yl]ceph-3-em-4-carboxylic acid,

(6R,7R)-7-[2-(2-aminothiazol-4-yl)-2-(Z)-methoxyiminoacetamido]-3-(1-methoxycarbonylcycloprop-1-yl)ceph-3-em-4-carboxylic acid, and

(6R,7R)-7-[2-(2-aminothiazol-4-yl)-2-(Z)-methoxyiminoacetamido]-3-(2RS)-1,1-bismethoxycarbonylcycloprop-2-yl]ceph-3-em-4-carboxylic acid.

7. A process for the preparation of a compound of formula (I) as claimed in any one of claims 1 to 6 which process comprises:

(a) treating a compound of formula (IV) or a salt thereof:

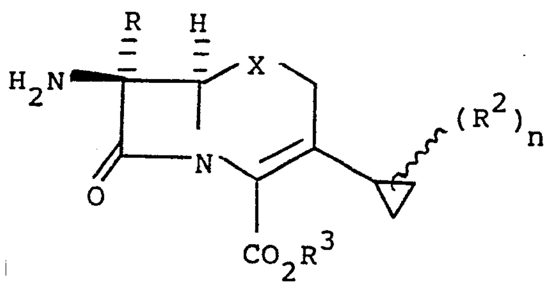

(IV)

wherein R, $R^2$, $R^3$, X and n are as defined with respect to formula (I) in claim 1 and wherein any reactive group may be protected, and wherein the amino group is optionally substituted with a group which permits acylation to take place; with an N-acylating derivative of an acid of formula (V):

$R^1OH$ (IV)

wherein $R^1$ is defined with respect to formula (I) in claim 1 and wherein any reactive groups may be protected; or

(b) heating an azetidinone compound of formula (VI):

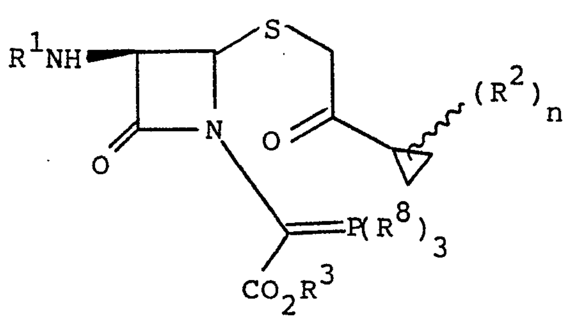

(VI)

wherein $R^1$, $R^2$, $R^3$ and n are as hereinbefore defined with respect to formula (I) in claim 1, $R^8$ is phenyl or n-butyl, and any reactive group may be protected; or

(c) heating a compound of formula (VIII):

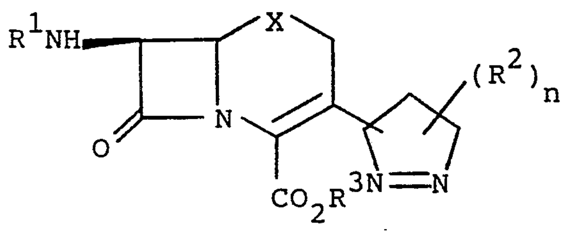

(VIII)

wherein $R^1$, $R^2$, $R^3$ and n are as hereinbefore defined with respect to formula (I) in claim 1, and any reactive group may be protected;
and thereafter if necessary or desired, carrying out one or more of the following steps:
(i) removing any protecting groups;
(ii) converting the group $R^3$ into a different group $R^3$;
(iii) converting the group $R^1$ into a different group $R^1$;
(iv) converting X into a different X;
(v) converting the product into a salt.

8. A compound of formula (IV) or a salt thereof:

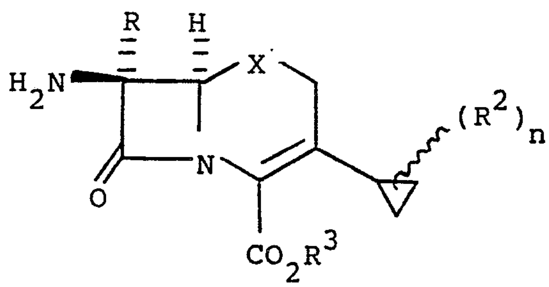

(IV)

wherein R, $R^2$, $R^3$, X and n are as defined with respect to formula (I) in claim 1.

9. A pharmaceutical composition comprising a compound of formula (Ia) or a pharmaceutically acceptable in vivo hydrolysable ester thereof, as claimed in claim 5 or claim 6 and a pharmaceutically acceptable carrier.

10. A compound of formula (Ia) or a pharmaceutically acceptable salt or a pharmaceutically acceptable in vivo hydrolysable ester thereof, as claimed in claim 5 or claim 6 for use as a therapeutic agent.